# EUROPEAN PATENT APPLICATION

(11) **EP 2 465 873 A1**
(43) Date of publication of application: **20.06.2012**
(21) Application number: 10075757.4
(22) Date of filing: 16.12.2010
(51) Int. Cl.: C07K 16/28

(54) **Eosinophils as a therapeutic target**

(71) Applicant: Deutsches Rheuma-Forschungszentrum Berlin, 10117 Berlin (DE)
(72) Inventor: Berek, Claudia, 12163 Berlin (DE); Van Trung Chu (VN), 10178 Berlin (DE)
(74) Representative: Lange, Sven

(57) **Abstract**

The present invention relates to a method for the depletion of plasma cells by depletion and/or functional inhibition of eosinophils. Another aspect of the present invention relates to an anti-eosinophil agent for use as a medicament for the depletion of plasma cells, preferably by induction of apoptosis. The invention also relates to a method for the treatment of an unwanted immune response, preferably an unwanted humoral immune response dependent on the numbers and/or activity of plasma cells, such as allergic reactions, autoimmune diseases and rejection of organs and/or tissue after transplantation, by administration of an anti-eosinophil agent according to the present invention, characterized in that plasma cells are depleted in the subject. A further aspect of the invention relates to a pharmaceutical composition comprising an anti-eosinophil agent according to the present invention, comprising preferably antibodies directed against Siglec-8 and/or IL-5, more preferably a combination of multiple antibodies directed against Siglec-8 and IL-5, with a pharmaceutically acceptable carrier.

## Description

The present invention relates to a method for the depletion of plasma cells by depletion and/or functional inhibition of eosinophils. Another aspect of the present invention relates to an anti-eosinophil agent for use as a medicament for the depletion of plasma cells, preferably by induction of apoptosis. The invention also relates to a method for the treatment of an unwanted immune response, preferably an unwanted humoral immune response dependent on the numbers and/or activity of plasma cells, such as allergic reactions, autoimmune diseases and rejection of organs and/or tissue after transplantation, by administration of an anti-eosinophil agent according to the present invention, characterized in that plasma cells are depleted in the subject. A further aspect of the invention relates to a pharmaceutical composition comprising an anti-eosinophil agent according to the present invention, comprising preferably antibodies directed against Siglec-8 and/or IL-5, more preferably a combination of multiple antibodies directed against Siglec-8 and IL-5, with a pharmaceutically acceptable carrier.

### BACKGROUND OF THE INVENTION

Memory B cells and plasma cells are the two arms of the humoral immune response that ensure long-term protection against pathogens¹⁻³. Plasma cells are essential contributors to this protection by secreting antigen-specific antibodies. Plasma cells can develop through different pathways⁴. Naive B cells challenged with antigen may directly differentiate into short-lived plasma cells, while long-lived plasma cells are generated predominantly in germinal centers during T cell-dependent immune responses⁵⁻⁶. Plasma blasts migrate to the bone marrow (BM) where they find an appropriate environment allowing them to differentiate into mature long-lived plasma cells⁷⁻⁹. During primary immune responses only a few antigen-specific plasma cells accumulate in the BM, though much higher numbers are found after secondary challenge with antigen¹⁰.

BM stromal cells provide a specific micro-environment that supports plasma cell survival through cell contact-dependent signals and cytokines^{4, 7, 11-12}. BM reticular stromal cells express CXCL12, a chemokine that both attracts plasma cells into the BM and supports their maintenance in this location¹³⁻¹⁵. In addition, cytokines such as APRIL (a proliferation-inducing ligand), interleukin-6 (IL-6), tumor necrosis factor (TNF)-α, IL-10, IL-4 and IL-5 are known to prolong the survival of plasma cells *in vitro*¹⁶. Although *in vitro* experiments suggested that IL-6 is a crucial cytokine for long-term plasma cell survival, the number of plasma cells is not reduced in the BM of IL-6-deficient mice^{11, 16}. In contrast, in mice deficient for APRIL, although the generation of plasma cells is normal, the capacity of the BM compartment to support plasma cell survival is strongly impaired¹⁷. Blocking APRIL by treatment of immunized animals with TACI-Ig (a fusion protein between the APRIL receptor TACI (transmembrane activator calcium modulator and cyclophilin ligand interactor) and human IgG) reduced the number of plasma cells in the BM by more than 60%¹⁸. Furthermore, mice deficient for the B cell maturation antigen (BCMA), a high affinity receptor of APRIL expressed by plasma cells, displayed significantly reduced numbers of plasma cells¹⁸. This suggests a critical role for APRIL in the long-term survival of plasma cells in the BM.

Little is known about the cellular sources of cytokines required for the survival of plasma cells *in vivo*^{11, 16-19}. In the mucosal-associated lymphoid tissue APRIL is locally produced by neutrophils and binds to heparin proteoglycans, hence providing a survival niche for plasma cells²⁰. Neutrophils infiltrating tumor lesions in the human BM constitutively produce APRIL²¹. In the murine lymph node, dendritic cells (DC) and, in particular, F4/80⁺Gr1^{lo} monocytes/macrophages (Mo/Mφ) are the main producers of APRIL and IL-6²².

Plasma cells are of crucial importance for long-term immune protection. It is thought that long-lived plasma cells survive in specialized niches in the bone marrow (BM). Plasma cells are also involved in unwanted immune responses, such as those involved in autoimmune diseases, tissue or organ transplantation rejections or allergic immune reactions. Until now few methods are available that effectively and selectively remove plasma cells in order to suppress or diminish unwanted immune reactions.

Plasma cell elimination is known to be an effective treatment of autoimmune diseases. Commonly used antibodies, such as Bortezomib are protease inhibitors and do not achieve a specific effect for plasma cells. The Bortezomib antibody is also toxic. These disadvantages demonstrate the need for developing novel approaches for reducing plasma cells, especially in bone marrow.

### SUMMARY OF THE INVENTION

The invention demonstrates that in BM eosinophils co-localize with plasma cells and are the key providers of plasma cell survival factors. In vitro, eosinophils support plasma cell survival by secreting APRIL and IL-6. In eosinophil-deficient mice, plasma cell numbers in BM are significantly reduced both at steady state and after immunization. Reconstitution experiments directly show that eosinophils are crucial for the retention of plasma cells in the BM. Moreover, depletion of eosinophils induces apoptosis in long-lived BM plasma cells.

The technical problem of the present invention can be seen as the provision of alternative means for plasma cell modulation that does not exhibit the disadvantages of the prior art.

The present invention therefore relates to a method for the modulation of plasma cell function and/or number by the modulation of eosinophil number and/or function.

In another embodiment the invention relates to the method described herein, characterised in that plasma cells are depleted by depletion and/or functional inhibition of eosinophils.

In another embodiment the invention relates to the method described herein, characterised in that the plasma cells and/or eosinophils are depleted via induction of apoptosis.

In another embodiment the invention relates to the method described herein, characterised in that IL-5, APRIL and/or IL-6 function, production and/or expression is inhibited.

In another embodiment the invention relates to the method described herein, characterised in that plasma cell function and/or numbers are induced and/or maintained by induction and/or functional activation of eosinophils.

In another embodiment the invention relates to the method described herein, characterised in that IL-5, APRIL and/or IL-6 function, production and/or expression is induced and/or activated.

In another embodiment the invention relates to the method described herein, characterised in that the plasma cells are antigen-specific plasma cells, preferably of the bone-marrow, epithelial, lung and/or intestinal tissues.

A further aspect of the invention relates to an eosinophil-modulating agent for use as a medicament for the modulation of plasma cell number and/or function.

In another embodiment the invention relates to the eosinophil-modulating agent as described herein, characterised in that the agent is an anti-eosinophil agent for use as a medicament for the depletion of plasma cells, preferably by induction of apoptosis. The invention therefore relates to an anti-eosinophil agent for use as a medicament for the depletion of plasma cells.

In another embodiment the invention relates to the anti-eosinophil agent as described herein for use as a medicament in the treatment of diseases characterised by an unwanted immune response.

In another embodiment the invention relates to the anti-eosinophil agent as described herein, whereby the disease is characterised by an unwanted humoral immune response, preferably dependent on the numbers and/or activity of plasma cells, such as allergic reactions, autoimmune diseases and rejection of organs and/or tissue after transplantation.

In another embodiment the invention relates to the anti-eosinophil agent as described herein for application in the lung, preferably in mucosal tissues of the lung, in the treatment of an allergic reaction, such as asthma.

In another embodiment the invention relates to the anti-eosinophil agent as described herein for reducing levels of IgE antibodies in the treatment of an unwanted inflammatory response.

In another embodiment the invention relates to the anti-eosinophil agent as described herein, characterised in that the agent prevents eosinophil development and/or facilitates depletion and/or inhibition of eosinophil function.

In another embodiment the invention relates to the anti-eosinophil agent as described herein, characterised in that the agent comprises one or more compounds that target eosinophils, specific antigens of eosinophils and/or molecules and/or signalling pathways important in eosinophil survival and/or development, such as Siglec-8 and/or IL-5.

A further aspect of the invention relates to an eosinophil-modulating agent as described herein, characterised in that the agent is an eosinophil-stimulating agent, such as IL-5, for use as a medicament for the induction and/or maintenance of plasma cells.

In another embodiment the eosinophil-modulating agent as described herein is characterised in that the agent is an antibody, small molecule, anti-sense nucleic acid molecule, such as siRNA, a cytokine and/or a peptide.

A further aspect of the invention relates to a pharmaceutical composition comprising an eosinophil-modulating agent, for example an eosinophil-stimulating or an anti-eosinophil agent, with a pharmaceutically acceptable carrier.

A further aspect of the invention relates to a pharmaceutical composition comprising an anti-eosinophil agent according to any one of claims 9 to 15, comprising preferably antibodies directed against Siglec-8 and/or IL-5, more preferably a combination of multiple antibodies directed against Siglec-8 and IL-5, with a pharmaceutically acceptable carrier.

A further aspect of the invention relates to a method for the treatment of an unwanted immune response, preferably an unwanted humoral immune response dependent on the numbers and/or activity of plasma cells, such as allergic reactions, autoimmune diseases and rejection of organs and/or tissue after transplantation, by administration of an anti-eosinophil as described herein to a subject, characterized in that plasma cells are depleted in the subject.

A further aspect of the invention relates to a method for determining the presence and/or numbers of plasma cells in a sample by measuring the presence and/or numbers of eosinophil cell numbers, whereby the presence and/or numbers of eosinophils is positively correlated with the presence and/or numbers of plasma cells.

### DETAILED DESCRIPTION OF THE INVENTION

The examples of the present invention show that eosinophils are the main source of plasma cell survival factors. In the BM they are found in close association with plasma cells, suggesting that eosinophils are part of the plasma cell survival niche. In eosinophil-deficient mice we see a significant reduction in the number of plasma cells both before and after antigen injection. In the BM of eosinophil-deficient ΔdblGATA-1 mice the frequency of plasma cells increased transiently when mice were reconstituted with eosinophils. This demonstrates that eosinophils are required for the retention and accumulation of plasma cells in the BM. In addition, the examples of the present invention disclose that eosinophils are required for the long-term survival of plasma cells in the BM as depletion of eosinophils drives long-lived plasma cells into apoptosis. The invention relates therefore to the pivotal role of eosinophils for the long-term maintenance of plasma cells in the BM.

A reduction in the numbers of and/or inhibition of the function of eosinophils leads directly to a reduction in the number of plasma cells in bone marrow. The present invention relates therefore to the use of eosinophils as a therapeutic target for reducing plasma cells. Eosinophils are required for maintaining plasma cells in bone marrow, especially in terms of long term plasma cell survival. The invention further relates to the inhibition and/or depletion of eosinophils, resulting in the reduction of plasma cells.

Plasma cells play a significant role in the humoral immune response, in addition to allergies, autoimmune diseases and rejection of tissue after transplantation. The invention therefore relates to eosinophils as a therapeutic target for the reduction of plasma cells in the treatment of diseases involved in immune responses.

The cytokine IL-5 is involved in the development and differentiation of eosinophils. The present invention therefore also relates to the suppression, depletion and/or inhibition of IL-5 for the reduction in numbers and/or function of eosinophils. The reduction of IL-5 also represents a therapeutic possibility for depleting eosinophil numbers in the treatment of diseases, in which immune response suppression would aid a therapeutic effect.

Also included in the scope of the invention are molecules which bind to eosinophils and lead to their suppression and/or destruction, such as antibodies, chelators and/or other binding agents. A preferred embodiment of the present invention relates to antibodies which target eosinophils. The invention relates to antibodies that target eosinophils and/or specific antigens thereof. The present invention also relates to antibodies targeted against Siglec proteins on the surface of eosinophils, such as Siglec-8, the human orthologue to Siglec-F, which is disclosed in this application. A further embodiment of the invention relates to the use of eosinophils or antigens thereof as target structures in the production of antibodies (either mono- or polyclonal, or fragments thereof), preferably for application in the methods of the present invention.

Additional target molecules included in the scope of the present invention that are important for the survival and/or development of eosinophils which can be targeted include GATA1, C/EBP-α, PU.1, FIP1L1-PDGFR, IL-5, IL-13, TSLP, Prostaglandin D2, Leukotriene B4, IL-5 receptor, common yc chain, α4 Integrins/VCAM-1, β7 Integrin/MAdCAM-1, β2 Integrins/ICAM-1, P-selectin, L-selectin, E-selectin, CCR3, CD33, CD44 and/or the Histamine H4 receptor. These molecules refer to the molecule names in the mouse. It requires no inventive effort on the part of a skilled practitioner to target the human homologues, orthologues and/or functional analogues of these molecules, so that the human counterparts are also included in the scope of the present invention.

Illnesses to be treated involve all diseases which would benefit from the elimination and/or suppression of plasma cells, such as diseases in which a suppression of the humoral immune system would be beneficial for therapeutic treatment, for example autoimmune diseases, tissue or organ transplantation rejections or allergic immune reactions. It is known that eosinophils produce an inflammation supporting effect.

Eosinophils are known to migrate, for example to the lungs, during allergic inflammatory reactions. The present invention therefore relates to the suppression and/or elimination of eosinophils in the lung, preferably the inflamed mucosal tissues, of a subject in the treatment of an allergic reaction, such as asthma. A further embodiment of the invention relates to the reduction of eosinophils in order to reduce the levels of IgE antibodies, which are involved in the inflammatory response.

The invention furthermore relates to a method of temporary reduction of plasma cells via elimination and/or depletion of eosinophils. This effect also leads to a reduction of plasma cells via the induction of apoptosis in plasma cells. The invention also relates to a method of inducing plasma cell apoptosis, preferably by administration of eosinophil-depleting agents. The invention also relates to a method for the treatment of unwanted humoral immune responses by reducing the number of and/or eliminating and/or suppressing eosinophils or eosinophil function.

The cytokines APRIL and IL-6 are produced by eosinophils and play an essential role in plasma cell maintenance. The method of reducing eosinophil numbers according to the invention can therefore be further characterised by reduction, suppression and/or inhibition of APRIL and/or IL-6 function, expression and/or production. The invention therefore relates to a method comprising the administration of soluble fusion protein TACI-Ig²⁵, and/or APRIL and/or IL-6 neutralizing antibodies as an embodiment of suppressing or minimizing eosinophil function. Although blocking of APRIL is known to reduce plasma cell numbers, there has been no disclosure in the prior art that eosinophils are related to his phenomena.

The cytokine IL-5 is required for the development of eosinophils. Although blocking of IL-5 is known to reduce eosinophil numbers, there has been no disclosure in the prior art that it effects plasma cell numbers.

A further aspect of the present invention relates to the induction and/or maintenance of plasma cell function and/or numbers by the application of eosinophils and/or the induction and/or stimulation of eosinophil function and/or development. A method for inducing plasma cell numbers is therefore also within the scope of the present invention, whereby an agent that stimulates eosinophil function or development leads to induction and/or maintenance of plasma cell function and/or numbers.

A further aspect of the present invention relates therefore to an eosinophil-stimulating agent, such as IL-5, for use as a medicament for the induction and/or maintenance of plasma cells. Such an eosinophil-stimulatng agent can either stimulate eosinophil function or the development of eosinophils in vivo, for example the triggering of eosinophil production.

The present invention therefore relates to an eosinophil-modulating agent, encompassing either anti-eosinophil agents, for the depletion of plasma cells, or eosinophil-stimulating agents, for the induction and/or maintenance of plasma cells. The invention relates to the dependence of plasma cells on eosinophils. According to this novel relationship between two cell types, the plasma cell number in a subject can be modulated, either enhanced or suppressed, via modulation of eosinophil number.

Eosinophils represent a novel target, which enable novel approaches towards the therapy of immune disorders.

An "eosinophil-modulating agent" relates to any substance that modulates eosinophil numbers, function, survival and/or development.

An "eosinophil-stimulating agent" relates to any substance that induces and/or maintains eosinophil function and/or numbers, or an agent that induces and/or stimulates eosinophil development.

An "anti-eosinophil agent" relates to any substance that leads to reduction of the number of eosinophils and/or eliminates eosinophils, preferably via induction of apoptosis, and/or suppresses eosinophils, eosinophil function and/or the development of eosinophils.

"Eosinophils" relate to white blood cells involved in immune responses, which are also known as eosinophil granulocytes. Eosinophils are defined by one or more of the following features: Strong eosin staining in the cytoplasm, expression of Siglec-F (in mice) or Siglec-8 (in humans) and/or expression of the following combination of markers: F4/80⁺Gr-1^{lo}CD11b^{int}Siglec-F^{hi}SSC^{hi}, or F4/80⁺Gr-1^{lo}CD11b^{hi}Siglec-F⁺SSC^{int}. These markers relate to the murine markers. Human eosinophils may be defined by expression of the analogous and/or orthologous human markers. A smaller size and bi-lobed nucleus with compact chromatin indicates mature eosinophils (Eos), whereas a larger size and doughnut or ring-shaped nucleus with loose chromatin indicates immature eosinophils (EosP).

"Plasma cells" relate to white blood cells that produce large amounts of antibodies, and are also known as plasma B cells, plasmocytes, or effector B cells. Plasma cells are defined by one or more of the following features: expression of CD138, Ig^{hi}, TACI, BCMA, FSC^{hi}, B220^{lo}, CXCR4, MHC classII^{lo}. These markers relate to the murine markers. Human plasma cells may be defined by expression of the analogous and/or orthologous human markers. CD38 and CD27 are markers for human plasma cells.

An "autoimmune disorder" or "autoimmune disease" herein is a disease or disorder arising from an immune response directed against an individual's own tissues or a co-segregate or manifestation thereof or resulting condition therefrom. Examples of autoimmune diseases or disorders include, but are not limited to arthritis (rheumatoid arthritis, juvenile-onset rheumatoid arthritis, osteoarthritis, psoriatic arthritis, and ankylosing spondylitis), psoriasis, dermatitis including atopic dermatitis, chronic idiopathic urticaria, including chronic autoimmune urticaria, polymyositis/ dermatomyositis, toxic epidermal necrolysis, scleroderma (including systemic scleroderma), sclerosis such as progressive systemic sclerosis, inflammatory bowel disease (IBD) (for example, Crohn's disease, ulcerative colitis, autoimmune inflammatory bowel disease), pyoderma gangrenosum, erythema nodosum, primary sclerosing cholangitis, episcleritis), respiratory distress syndrome, including adult respiratory distress syndrome (ARDS), meningitis, IgE-mediated diseases such as anaphylaxis and allergic and atopic rhinitis, encephalitis such as Rasmussen's encephalitis, uveitis or autoimmune uveitis, colitis such as microscopic colitis and collagenous colitis, glomerulonephritis (GN) such as membranous GN (membranous nephropathy), idiopathic membranous GN, membranous proliferative GN (MPGN), including Type I and Type II, and rapidly progressive GN, allergic conditions, allergic reaction, eczema, asthma, conditions involving infiltration of T cells and chronic inflammatory responses, atherosclerosis, autoimmune myocarditis, leukocyte adhesion deficiency, systemic lupus erythematosus (SLE) such as cutaneous SLE, subacute cutaneous lupus erythematosus, lupus (including nephritis, cerebritis, pediatric, non-renal, discoid, alopecia), juvenile onset (Type I) diabetes mellitus, including pediatric insulin-dependent diabetes mellitus (IDDM), adult onset diabetes mellitus (Type II diabetes), multiple sclerosis (MS) such as spino-optical MS, immune responses associated with acute and delayed hypersensitivity mediated by cytokines and T-lymphocytes, tuberculosis, sarcoidosis, granulomatosis including lymphomatoid granulomatosis, Wegener's granulomatosis, agranulocytosis, vasculitis (including large vessel vasculitis (including polymyalgia rheumatica and giant cell (Takayasu's) arteritis), medium vessel vasculitis (including Kawasaki's disease and polyarteritis nodosa), CNS vasculitis, systemic necrotizing vasculitis, and ANCA-associated vasculitis, such as Churg-Strauss vasculitis or syndrome (CSS)), temporal arteritis, aplastic anemia, Coombs positive anemia, Diamond Blackfan anemia, hemolytic anemia or immune hemolytic anemia including autoimmune hemolytic anemia (AIHA), pernicious anemia, pure red cell aplasia (PRCA), Factor VIII deficiency, hemophilia A, autoimmune neutropenia, pancytopenia, leukopenia, diseases involving leukocyte diapedesis, CNS inflammatory disorders, multiple organ injury syndrome, antigen-antibody complex mediated diseases, anti-glomerular basement membrane disease, antiphospholipid antibody syndrome, allergic neuritis, Bechet's or Behcet's disease, Castleman's syndrome, Goodpasture's syndrome, Reynaud's syndrome, Sjogren's syndrome, Stevens-Johnson syndrome, pemphigoid such as pemphigoid bullous, pemphigus (including vulgaris, foliaceus, and pemphigus mucus-membrane pemphigoid), autoimmune polyendocrinopathies, Reiter's disease, immune complex nephritis, chronic neuropathy such as IgM polyneuropathies or IgMmediated neuropathy, thrombocytopenia (as developed by myocardial infarction patients, for example), including thrombotic thrombocytopenic purpura (TTP) and autoimmune or immune-mediated thrombocytopenia such as idiopathic thrombocytopenic purpura (ITP) including chronic or acute ITP, autoimmune disease of the testis and ovary including autoimune orchitis and oophoritis, primary hypothyroidism, hypoparathyroidism, autoimmune endocrine diseases including thyroiditis such as autoimmune thyroiditis, chronic thyroiditis (Hashimoto's thyroiditis), or subacute thyroiditis, autoimmune thyroid disease, idiopathic hypothyroidism, Addison's disease, Grave's disease, polyglandular syndromes such as autoimmune polyglandular syndromes (or polyglandular endocrinopathy syndromes), paraneoplastic syndromes, including neurologic paraneoplastic syndromes such as Lambert-Eaton myasthenic syndrome or Eaton-Lambert syndrome, stiff-man or stiff-person syndrome, encephalomyelitis such as allergic encephalomyelitis, myasthenia gravis, cerebellar degeneration, limbic and/or brainstem encephalitis, neuromyotonia, opsoclonus or opsoclonus myoclonus syndrome (OMS), and sensory neuropathy, Sheehan's syndrome, autoimmune hepatitis, chronic hepatitis, lupoid hepatitis, chronic active hepatitis or autoimmune chronic active hepatitis, lymphoid interstitial pneumonitis, bronchiolitis obliterans (non-transplant) vs NSIP, GuillainBarre syndrome, Berger's disease (IgA nephropathy), primary biliary cirrhosis, celiac sprue (gluten enteropathy), refractory sprue, dermatitis herpetiformis, cryoglobulinemia, amyotrophic lateral sclerosis (ALS; Lou Gehrig's disease), coronary artery disease, autoimmune inner ear disease (AIED) or autoimmune hearing loss, opsoclonus myoclonus syndrome (OMS), polychondritis such as refractory polychondritis, pulmonary alveolar proteinosis, amyloidosis, giant cell hepatitis, scleritis, a non-cancerous lymphocytosis, a primary lymphocytosis, which includes monoclonal B cell lymphocytosis (e.g., benign monoclonal gammopathy and monoclonal gammopathy of undetermined significance, MGUS), peripheral neuropathy, paraneoplastic syndrome, channelopathies such as epilepsy, migraine, arrhythmia, muscular disorders, deafness, blindness, periodic paralysis, and channelopathies of the CNS, autism, inflammatory myopathy, focal segmental glomerulosclerosis (FSGS), endocrine ophthalmopathy, uveoretinitis, autoimmune hepatological disorder, fibromyalgia, multiple endocrine failure, Schmidt's syndrome, adrenalitis, gastric atrophy, presenile dementia, demyelinating diseases, Dressler's syndrome, alopecia areata, CREST syndrome (calcinosis, Raynaud's phenomenon, esophageal dysmotility, sclerodactyly, and telangiectasia), male and female autoimmune infertility, ankylosing spondolytis, mixed connective tissue disease, Chagas' disease, rheumatic fever, recurrent abortion, farmer's lung, erythema multiforme, post-cardiotomy syndrome, Cushing's syndrome, bird-fancier's lung, Alport's syndrome, alveolitis such as allergic alveolitis and fibrosing alveolitis, interstitial lung disease, transfusion reaction, leprosy, malaria, leishmaniasis, kypanosomiasis, schistosomiasis, ascariasis, aspergillosis, Sampter's syndrome, Caplan's syndrome, dengue, endocarditis, endomyocardial fibrosis, endophthalmitis, erythema elevatum et diutinum, erythroblastosis fetalis, eosinophilic faciitis, Shulman's syndrome, Felty's syndrome, flariasis, cyclitis such as chronic cyclitis, heterochronic cyclitis, or Fuch's cyclitis, Henoch-Schonlein purpura, human immunodeficiency virus (HIV) infection, echovirus infection, cardiomyopathy, Alzheimer's disease, parvovirus infection, rubella virus infection, post-vaccination syndromes, congenital rubella infection, Epstein-Barr virus infection, mumps, Evan's syndrome, autoimmune gonadal failure, Sydenham's chorea, post-streptococcal nephritis, thromboangitis ubiterans, thyrotoxicosis, tabes dorsalis, and giant cell polymyalgia. The treatment of autoimmune disorders via immune suppression is therefore one embodiment of the present invention.

As used herein, the term "allergy" or "allergies" or "allergic reaction" or "allergic response" refers to a disorder (or improper reaction) of the immune system. Allergic reactions occur to normally harmless environmental substances known as allergens; these reactions are acquired, predictable, and rapid. Strictly, allergy is one of four forms of hypersensitivity and is called type I (or immediate) hypersensitivity. It is characterized by excessive activation of certain white blood cells called mast cells and basophils by a type of antibody known as IgE, resulting in an extreme inflammatory response. Common allergic reactions include eczema, hives, hay fever, asthma, food allergies, and reactions to the venom of stinging insects such as wasps and bees. Mild allergies like hay fever are highly prevalent in the human population and cause symptoms such as allergic conjunctivitis, itchiness, and runny nose. Allergies can play a major role in conditions such as asthma. In some people, severe allergies to environmental or dietary allergens or to medication may result in life-threatening anaphylactic reactions and potentially death. The treatment of allergies via immune suppression is therefore one embodiment of the present invention.

As used herein "pharmaceutical composition" means a composition comprising an anti-eosinophil agent and at least one ingredient that is not an active ingredient whereby the composition can be safely and effectively used as a product to obtain or achieve a desired outcome. The term "pharmaceutical composition" as used herein means compositions which result from the combination of individual components which are themselves pharmaceutically acceptable. For example, where intravenous administration is foreseen, the components are suitable or acceptable (in both quality and quantity) for intravenous administration. The anti-eosinophil agent of the present invention can be administered to mammals, namely humans and livestock, by numerous routes, such as intravenously, subcutaneously or intramuscularly. The dose administered is understood by a person of ordinary skill in the art to be a therapeutically effective dose, when considering a therapy to treat symptoms of unwanted immune reactions. The disclosure presented herein is directed towards a pharmaceutical composition which can be administered through a variety of routes including intravenously, subcutaneously, intramuscularly or directly into or onto the affected individual. When the pharmaceutical composition is delivered via an injection, the injection of the anti-eosinophil agent composition can occur as a single injection or multiple injections at any location inside or outside the body and the injection(s) can occur in a single day or over multiple days. The daily dose is administered to a subject wherein the daily amount of the anti-eosinophil agent delivered to the subject from the pharmaceutical composition is about that which is therapeutically effective for treating symptoms associated with unwanted immune reactions. Additionally, the pharmaceutical composition may optionally include additional components such as salts, stabilizers and antimicrobials without departing from the spirit and scope of the claimed invention. The pharmaceutical composition of the present invention contains an anti-eosinophil agent preparation and a pharmaceutically acceptable carrier. The quantity and nature of the anti-eosinophil agent to be incorporated in the composition will vary depending on desired therapeutic effect and the time span for which the composition is to provide a therapeutic effect. The quantity of anti-eosinophil agent in the pharmaceutical composition is that which will deliver a therapeutically effective amount for treating symptoms associated with unwanted immune reactions. Of course, the concentration and character of the anti-eosinophil agent to be included in the pharmaceutical composition will vary depending upon the components used in the composition, the route by which it is administered, the symptoms and details of the immune reaction which requires treatment as well as other factors known to those of skill in the art.

Although the invention has been described with respect to specific embodiments and examples, it should be appreciated that other embodiments utilizing the concept of the present invention are possible without departing from the scope of the invention. The present invention is defined by the claimed elements, and any and all modifications, variations, or equivalents that fall within the true spirit and scope of the underlying principles.

### Description of the Figures

- Figure 1:: BM eosinophils provide plasma cell survival factors.
- Figure 2:: Eosinophils support plasma cell survival through secretion of soluble factors.
- Figure 3:: In the BM eosinophils and plasma cells colocalize.
- Figure 4:: The level of APRIL and IL-6 expression is reduced in the BM of eosinophil-deficient mice.
- Figure 5:: Plasma cell accumulation is impaired in the BM of eosinophil-deficient mice.
- Figure 6:: Eosinophils are required for the retention of plasma cells in the BM.
- Figure 7:: The long-term survival of plasma cells is dependent on eosinophils.
- Figure 8:: Sorting BM cell subsets.
- Figure 9:: *Ex vivo* cytokine expression in BM cell subsets.
- Figure 10:: Activation of BM eosinophils *in vivo.*
- Figure 11:: Eosinophils support plasma cell survival through secretion of soluble factors.
- Figure 12:: BM eosinophils do not produce CXCL12, but express CXCR4.
- Figure 13:: The level of APRIL and IL-6 expression is reduced in the BM of PHIL mice.
- Figure 14:: Plasma cell accumulation in the BM is impaired in eosinophil-deficient mice.
- Figure 15:: B cell development in ΔdbIGATA-1 mice is normal.
- Figure 16:: BM architecture and CXCR4 chemokine receptor expression are normal in ΔdblGATA-1 mice.
- Figure 17:: Normal initial immune response in ΔdbIGATA-1 mice.
- Figure 18:: Eosinophil-deficiency affects numbers of plasma cells in spleen of LCMV infected mice.

### Detailed description of the Figures

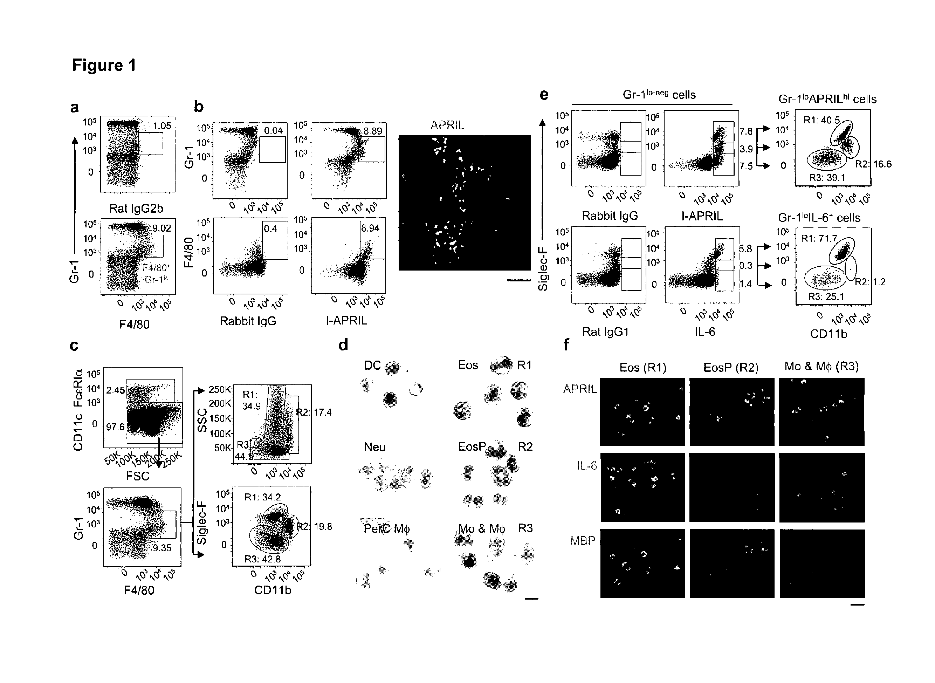

**Figure 1****: BM eosinophils provide plasma cell survival factors. (a)** Percentage of F4/80⁺Gr-1^{lo} in the BM. **(b)** Left, APRIL intracellular staining of Gr-1^{lo} (upper) and F4/80⁺ (lower row) BM cells. Right, immunohistology showing colocalization of APRIL and F4/80 stains in the BM. Scale bar 75µm. **(c)** Flow cytometry displaying the identification of 3 different CD11b⁺ subsets (R1, R2 and R3) among CD11c⁻FcεRIα⁻Gr-1^{neg/lo}(Gr-1^{N lo}) cells. **(d)** Hematoxylin/eosin staining of BM cell subsets sorted and prepared by cytospins. Peritoneal cavity (PerC) Mφ were used as controls. DC, neutrophils (Neu), eosinophils (Eos), immature eosinophils (EosP). Scale bar 15µm. **(e)** Analysis of intracellular expression of APRIL (upper row) or IL-6 (lower row) in BM cells gated on Gr-1^{N lo} cells. Right panels show the percentage of R1, R2 and R3 cells in the fraction of Gr-1^{lo}APRIL^{hi} or Gr-1^{lo}IL-6⁺ cells. **(f)** Immunoflourescence of sorted eosinophils (Eos, R1), immature eosinophils (EosP, R2) and Mo/Mφ (R3) stained with APRIL, IL-6 and MBP specific antibodies. Scale bar 45µm. Nuclei were counterstained with 4-6-diamidino-2-phenylindole (DAPI). Each panel is representative of 3 to 5 experiments.

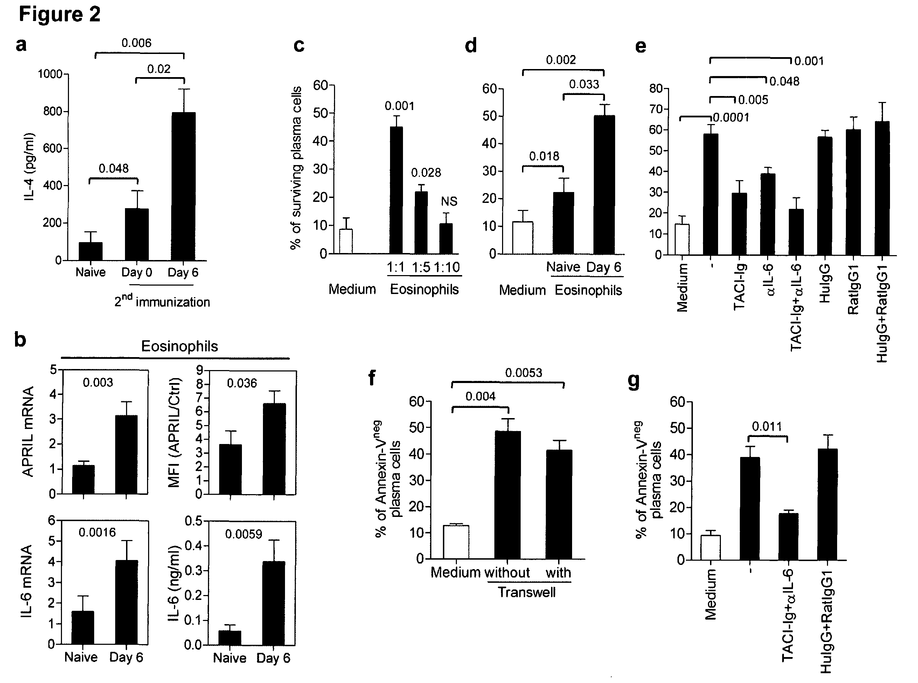

**Figure 2****: Eosinophils support plasma cell survival through secretion of soluble factors. (a)** IL-4 secretion by eosinophils from the BM of naive and immunized BALB/c (day 0 and 6 of secondary (2°) response). **(b)** Relative units (RU) of APRIL and IL-6 mRNA, MFI of intracellular APRIL staining and amount of IL-6 secretion is shown. 4 mice per group were analyzed. **(c)** Plasma cells were cultured with medium (Med, □) or different numbers of eosinophils (Eos (■), day 6 of secondary response). The percentage of plasma cells (PC) was determined by ELISPOT. **(d)** Plasma cells were cultured (ratio 1:1) with eosinophils from naïve or secondary response (day 6). **(e)** Plasma cells were cocultured with eosinophils (day 6, ratio 1:1) and inhibitors added as indicated. **(f)** Plasma cells were cultured with eosinophils (day 6, ratio 1:1) in transwells with (+) or without inserts (N). **(g)** Inhibitors were added as indicated and the percentage of Annexin-V^{neg} (AnnV^{N}) plasma cells determined. Results of triplicate cultures are summarized. One of two experiments is shown. P values or NS (no significance) are indicated, and error bars indicate s.d.

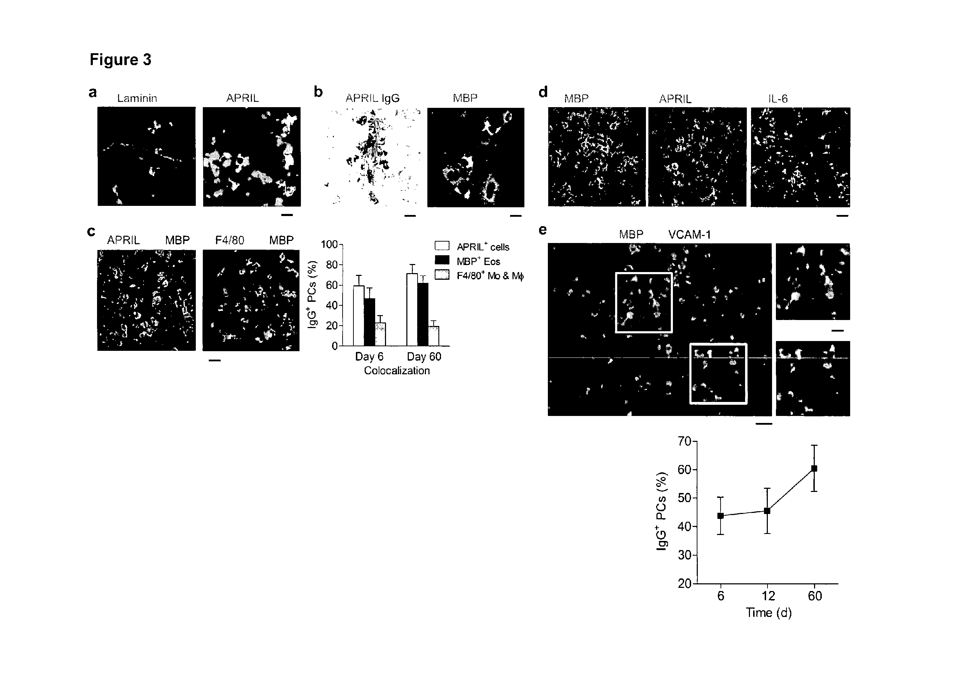

**Figure 3****: In the BM eosinophils and plasma cells colocalize. (a)** BM sections from day 6 of secondary response were double stained with fluorescence labeled antibodies. Overlays are shown. Scale bar 15µm. **(b)** BM sections from day 60 of secondary response. Left, immunohistochemical staining (scale bar 20µm), right, immunofluorescence staining analyzed by confocal microscopy (scale bar 10µm). **(c)** BM tissue sections from day 6 of secondary response triple stained with plasma cell (IgG), eosinophil (MBP) and APRIL or Mo/Mφ (F4/80) specific antibodies. Scale bar 20µm. Graph shows the percentage of IgG⁺ plasma cells (IgG⁺ PCs) colocalizing with APRIL⁺, MBP⁺ and F4/80⁺ cells. **(d)** BM tissue sections from day 6 of the secondary response stained for VCAM-1 and MBP (left), MBP and APRIL (middle), MBP and IL-6 (right). Scale bar 20µm. **(e)** BM tissue section from day 6 of secondary response (scale bar 75µm) stained with VCAM-1, IgG and MBP specific antibodies. Inlets show higher magnification. Representative data from five immunized animals are shown. Graph shows the percentage of IgG⁺ PCs colocalizing with MBP⁺ eosinophils and VCAM-1⁺ stromal cells days (d) after secondary immunization. Results from five animals per group are summarized. Error bars indicate s.d.

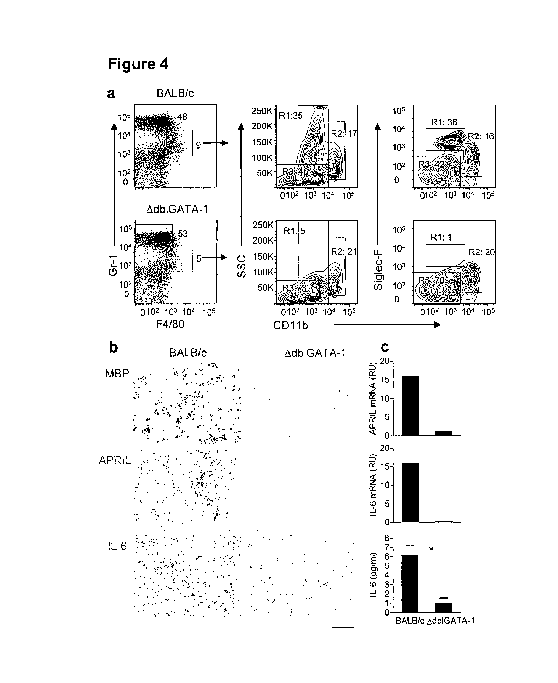

**Figure 4****: The level of APRIL and IL-6 expression is reduced in the BM of eosinophil-deficient mice. (a)** Gating on BM F4/80⁺Gr-1^{lo} cells the percentage of eosinophils (R1), immature eosinophils (R2) and Mo/Mφ (R3) is shown for BALB/c and ΔdblGATA-1 mice. **(b)** Immunohistological stainings of BM tissue sections from BALB/c and ΔdblGATA-1 mice for MBP (upper), APRIL (middle) and IL-6. Scale bar 150µm. **(c)** Mean values for the relative units (RU) of APRIL (upper) and IL-6 mRNA (middle) and the amount of IL-6 secretion (lower) in total BM cells are given. Representative data for 4 animals are shown. P values and error bars indicate s.d.

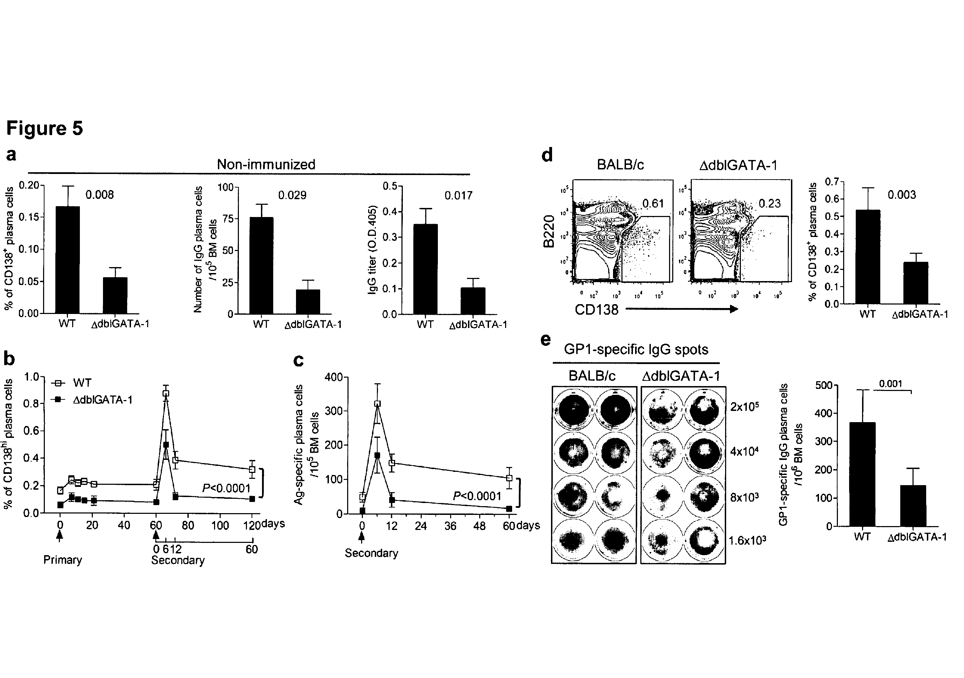

**Figure 5****: Plasma cell accumulation is impaired in the BM of eosinophil-deficient mice. (a)** The percentage of CD138⁺ plasma cells (left), the number of IgG plasma cells (IgG⁺ PC) (middle) and IgG secretion (A₄₀₅) was determined (right) for total BM cells of non-immunized BALB/c (n=3) and ΔdblGATA-1 (n=4) mice. **(b)** The percentage of CD138^{hi} and **(c)** antigen-specific plasma cells in the BM of phOx-immunized BALB/c and ΔdblGATA-1 mice is shown. 4 animals per group were analyzed at each time point of the primary (1°) and secondary (2°) response. Plasma cells in the BM of LCMV infected BALB/c and ΔdblGATA-1 mice are shown. **(d)** The percentage of CD138⁺ plasma cells is shown by contour plots, the block diagram summarizes data. **(e)** The number of GP1-specific IgG⁺ plasma cells is shown by ELISPOT, the block diagram summarizes data. 6 animals per group were analyzed. P values are indicated. All error bars are s.d.

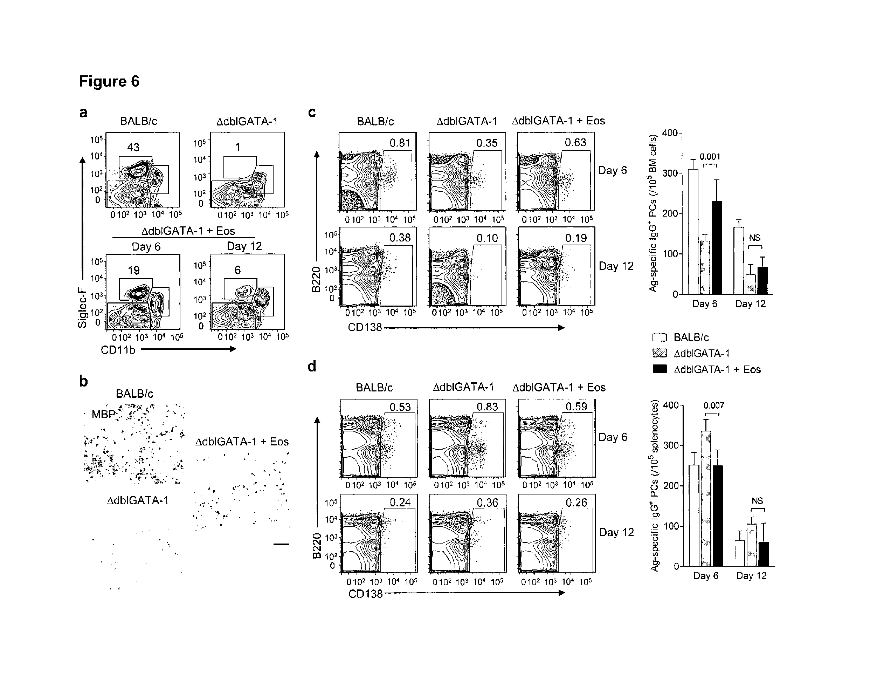

**Figure 6****: Eosinophils are required for the retention of plasma cells in the BM.** Data from BALB/c, ΔdblGATA-1 and ΔdblGATA-1 mice reconstituted with eosinophils (ΔdblGATA-1 + Eos) are shown. **(a)** Flow cytometry displaying the frequency of Siglec-F⁺CD11b^{int} eosinophils and Siglec-F^{int}CD11b^{hi} immature eosinophils. **(b)** MBP staining of BM tissue sections determining the number of eosinophils. Scale bar 150µm. Representative results from one of 3 animals per group are given. (c) Left, contour plots showing the percentage of CD138⁺ plasma cells, right, block diagram showing the number of antigen (Ag)-specific IgG plasma cells (IgG⁺ PC) in the BM 6 and 12 days after secondary immune response. Three animals per group were analyzed. **(d)** represents data obtained from the spleen of these animals. P values or NS (no significance) are indicated. All error bars are s.d.

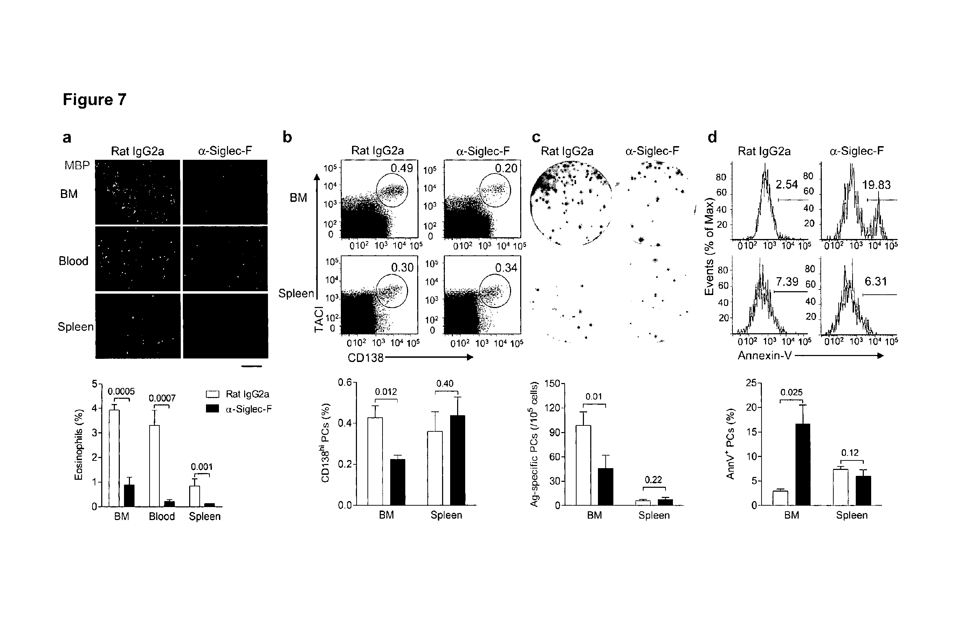

**Figure 7****: The long-term survival of plasma cells is dependent on eosinophils. (a)** The number of MBP⁺ eosinophils in tissue sections from BM (upper) and spleen (lower) and in cytospins from blood samples (middle) of control (Rat IgG2a) or eosinophil depleted BALB/c mice (α-Siglec-F). Scale bar 300µm. 3 animals per group were analyzed. Data are summarized in the block diagram. **(b)** The percentage of CD138⁺TACI⁺ plasma cells, **(c)** the absolute number of antigen- specific plasma cells and **(d)** the frequency of apoptotic plasma cells (AnnV⁺) in BM (upper row) and spleen (lower row). Six animals per group were analyzed. Data are summarized in block diagrams. P values are indicated. All error bars are s.d.

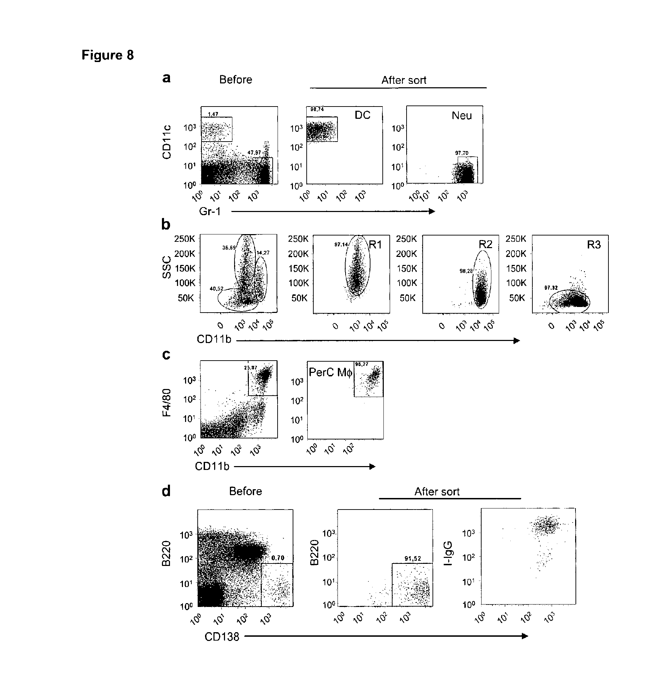

**Figure 8****: Sorting BM cell subsets. (a)** To isolate dentritic cells (DC), BM cells were stained with anti-CD11c-PE, incubated with anti-PE microbeads, enriched by MACS and sorted by FACS. The negative fraction was stained with anti-Gr-1 and anti-CD11b and neutrophils (Neu) sorted. **(b)** To isolate R1 (eosinophils), R2 (immature eosinophils) and R3 (Mo/Mφ) cells, BM suspension was depleted of B and T cells by MACS and cells stained with anti-CD11c/FcεRlα, anti-Gr-1, anti-F4/80 and anti-CD11b. DC, basophils/mast cells and Gr-1^{hi} cells were excluded by gating. The R1, R2 and R3 cell subsets were sorted based on SSC and on their expression levels of CD11b. (c) Peritoneal cavity cells were double stained with F4/80 and CD11 b specific antibodies and Mφ (PerC-Mφ) enriched. **(d)** Plasma cells were sorted and purity controlled by staining with anti-B220 or intracellular IgG.

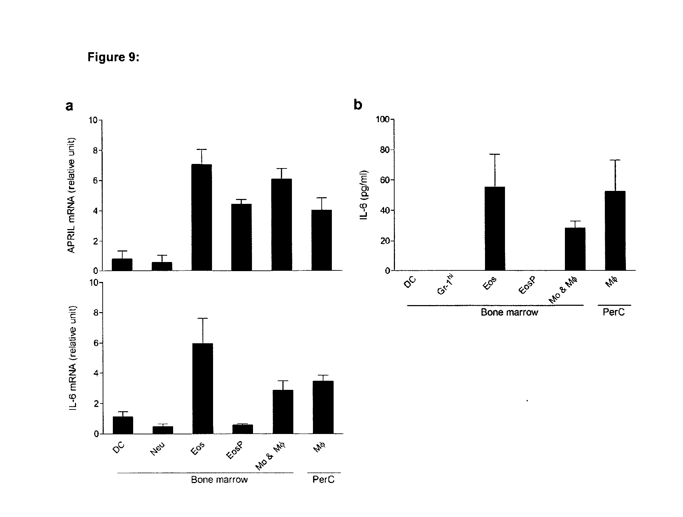

**Figure 9****: *Ex vivo* cytokine expression in BM cell subsets. (a)** The different BM cell subsets were sorted, mRNA prepared and the level of APRIL and IL-6 mRNA compared by real-time PCR. The expression levels were normalized to that of ß-actin. **(b)** Sorted BM cell subsets were cultured for 24h and constitutive IL-6 expression determined. The results from 3 independent experiments are shown. All error bars are s.d.

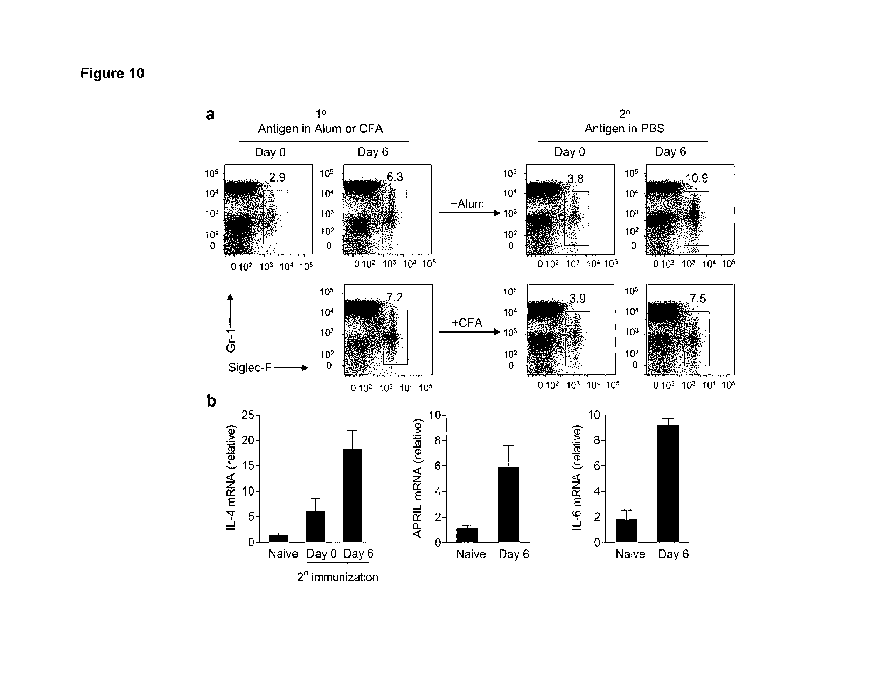

**Figure 10****: Activation of BM eosinophils *in vivo.* (a)** BALB/c mice were immunized i.p. with Alum-precipitated or CFA-emusified phOx-CSA (1° immunization) and 8 weeks later boosted i.v. with soluble antigen (2° immunization). At indicated time points BM cells were stained with antibodies specific for Gr-1 and Siglec-F. The percentage of eosinophils is shown as dot plots. **(b)** BALB/c mice were immunized i.p. with CFA-emusified phOx-CSA and 8 weeks later boosted i.v. with soluble antigen. Eosinophils were sorted from the BM of naive BALB/c and at days 0 and 6 of secondary immunization. The relative amount of IL-4, APRIL and IL-6 mRNA was determined. 4 mice per group were analyzed. All error bars are s.d.

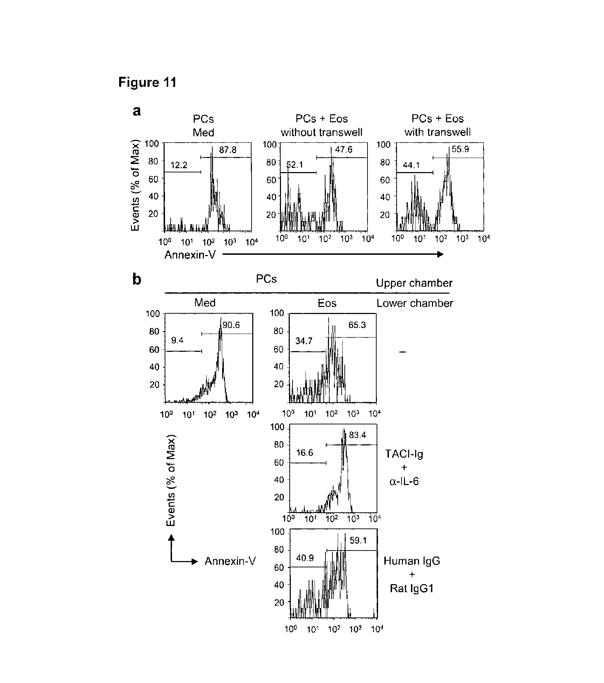

**Figure 11****: Eosinophils support plasma cell survival through secretion of soluble factors.**
**(a)** 10⁴ BM plasma cells were cultured with medium (Med) or eosinophils (Eos, ratio 1:1) in transwells with or without inserts. **(b)** Inhibitors were added as indicated. Human IgG and rat IgG1 were used as isotype controls. After 48h culture cells were stained with anti-CD138 and Annexin-V and analyzed by flow cytometry. The percentage of apoptotic and Annexin-V^{neg} surviving cells was determined. A representative result from 2 similar experiments is shown.

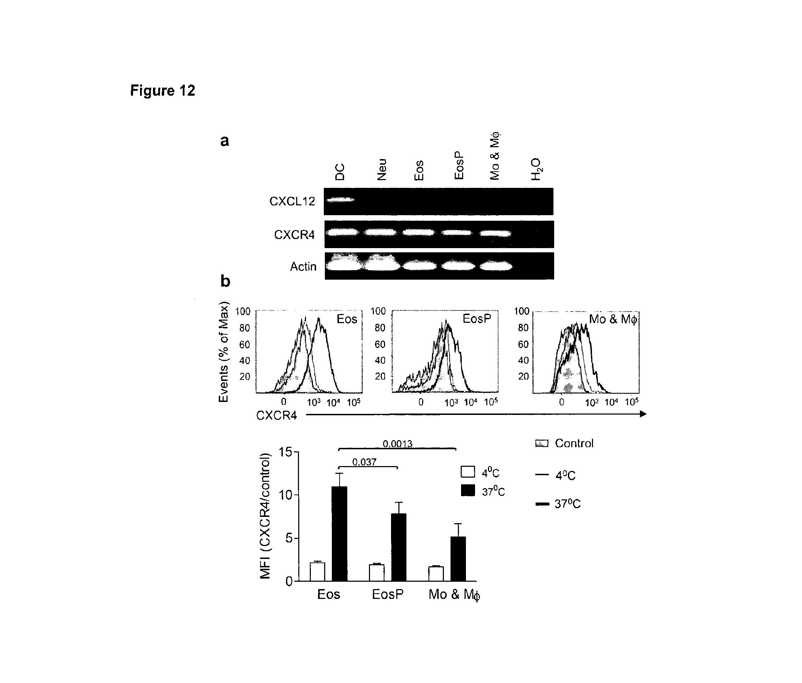

**Figure 12****: BM eosinophils do not produce CXCL12, but express CXCR4. (a)** BM cell subsets were sorted and CXCL12 and CXCR4 transcripts determined by RT-PCR. **(b)** CXCR4 surface expression for eosinophils (Eos), immature eosinophils (EosP) and Mo & Mφ was analyzed by flow cytometry. Histograms show overlays of isotype control (grey), surface expression at 4°C (normal line) or after incubation at 37°C (heavy line). MFI for CXCR4 expression was determined at 4° (□) and 37° (■). P values are indicated and error bars indicate s.d.

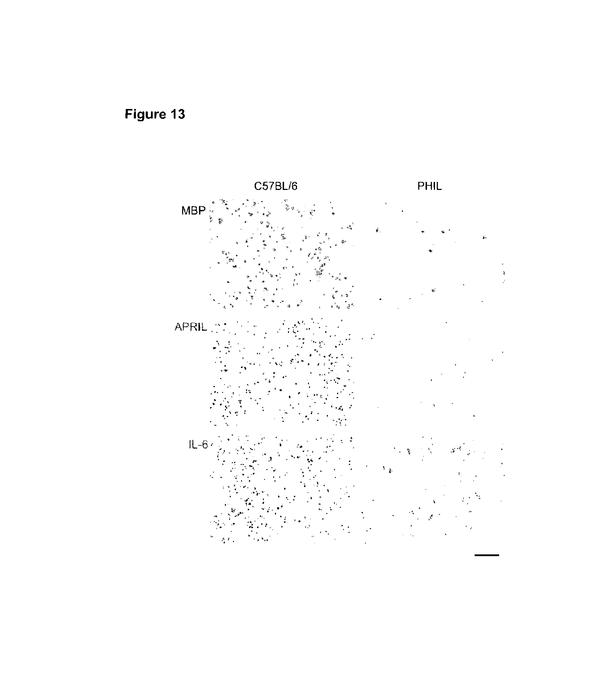

**Figure 13****: The level of APRIL and IL-6 expression is reduced in the BM of PHIL mice.** BM tissue sections of C57BL/6 and PHIL mice were immunohistochemically stained with antibodies specific for MBP, APRIL and IL-6. Scale bar 150 µm.

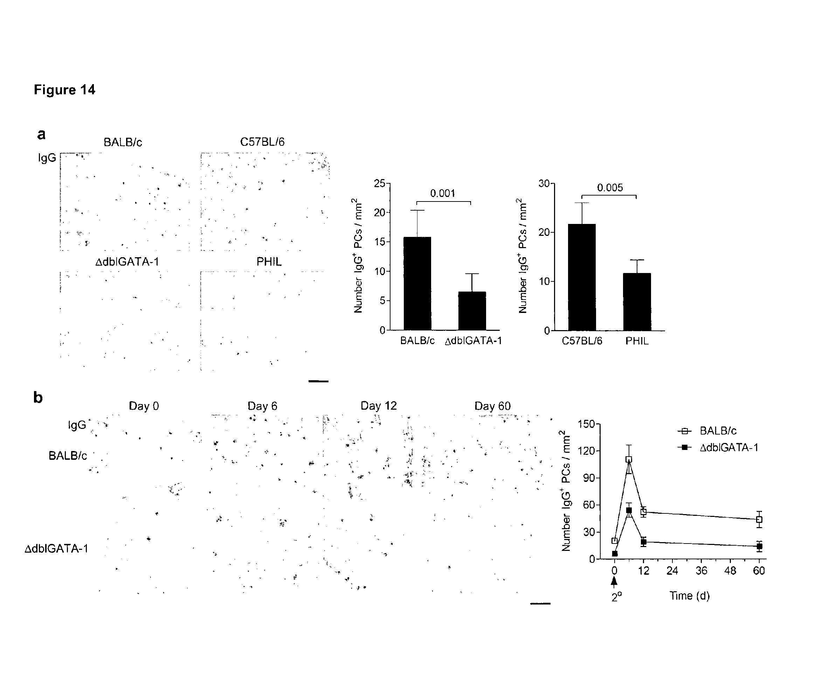

**Figure 14****: Plasma cell accumulation in the BM is impaired in eosinophil-deficient mice.** BM sections were prepared from **(a)** naive BALB/c (n=4), C57BL/6 (n=2), ΔdbIGATA-1 (n=4) and PHIL (n=2) mice and (b) from BALB/c (□) and ΔdbIGATA-1 (■) mice at different time points after secondary immunization. Sections were stained with anti-mouse IgG and the number of plasma cells counted. For secondary immunization 3 animals per group were analyzed at each time point, data summarized and presented as graph. Scale bar **(a)** 300 µm and **(b)** 150 µm. P values are shown and error bars indicate s.d.

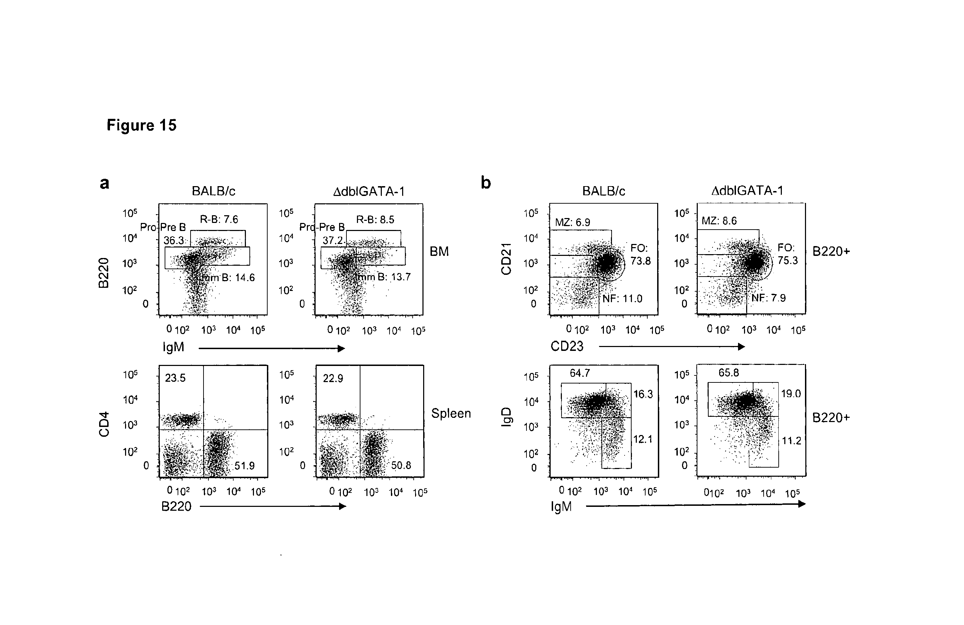

**Figure 15****: B cell development in ΔdbIGATA-1 mice is normal. (a)** BM and spleen cell suspensions were stained with anti-B220 and IgM or CD4, respectively. Gating on living lymphocytes showed that the percentage of pro-pre, immature (Imm), re-circulating (R) B cell subsets in BM of ΔdbIGATA-1 mice is normal. In the spleen the ratio of CD4 T cells and B cells are normal. (b) Splenocytes were stained with for B220, CD21, CD23, IgM and IgD. Gating on B220 showed normal development of newly formed (NF) T1 and T2, marginal zone (MZ) and follicular (FO) B cells. Representative data from one of 4 animals are shown.

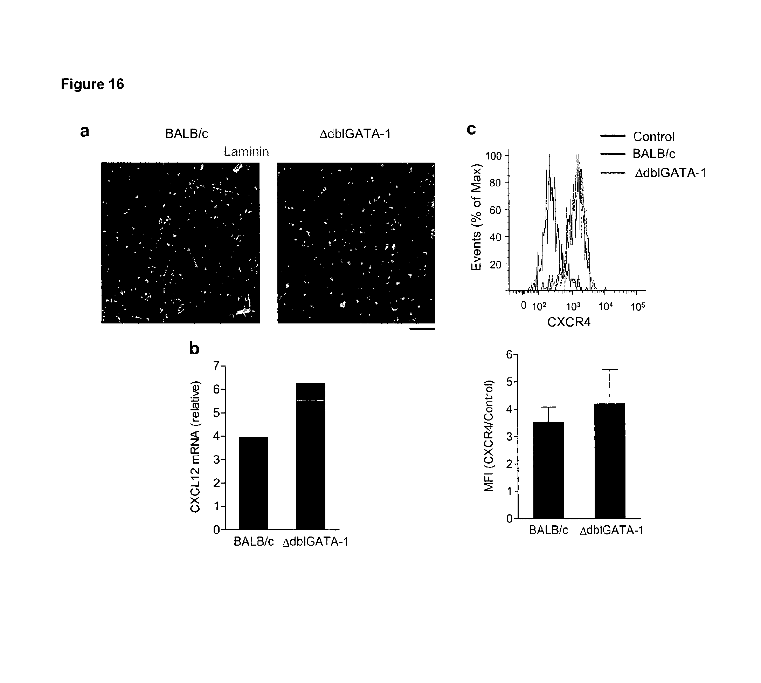

**Figure 16****: BM architecture and CXCR4 chemokine receptor expression are normal in ΔdbIGATA-1 mice. (a)** BM sections of BALB/c and ΔdbIGATA-1 mice were stained with anti-Laminin (red) and anti-VCAM-1 (green). Scale bar 75 µm. **(b)** The expression level of BM CXCL12 mRNA was compared by real-time PCR. The expression levels were normalized to that of ß-actin, mean values are shown. Representative data for one of 4 animals are shown. (c) A histogram shows overlays of CXCR4 expression on the surface of splenic CD138⁺ plasma cells from BALB/c (blue), ΔdbIGATA-1 (red) and isotype control (black) at day 6 of secondary immunization. Ratio of MFI of CXCR4 expression on plasma cells to the MFI of the isotype control is shown. 4 animals per group were analyzed. All error bars are s.d.

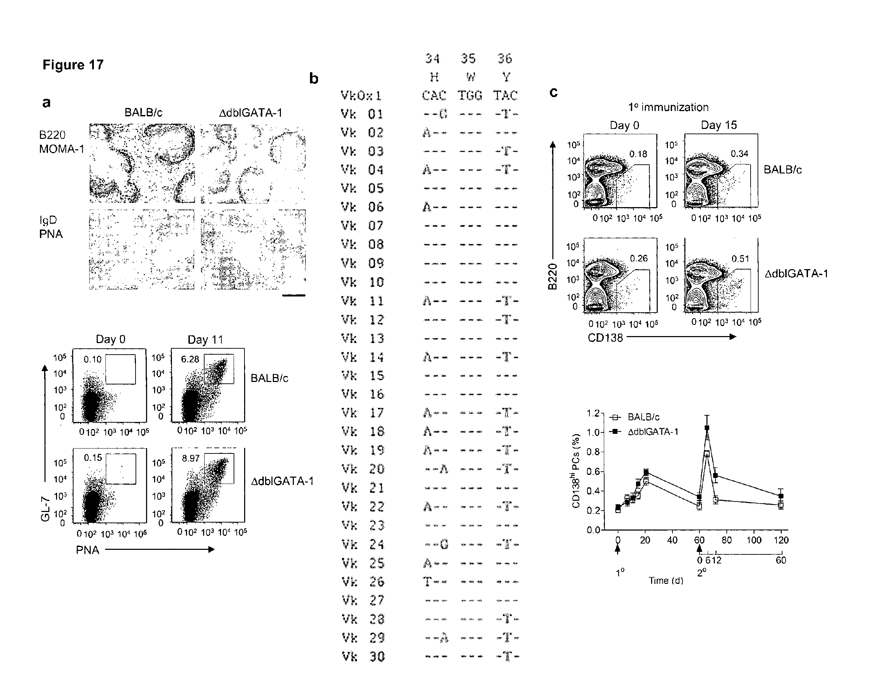

**Figure 17****: Normal initial immune response in ΔdbIGATA-1 mice.** BALB/c and ΔdbIGATA-1 mice were immunized i.p. with phOx-CSA in alum. **(a)** Staining of splenic tissue sections with B220 and MOMA-1 or anti-IgD and PNA shows normal lymphoid structures and GC development. Scale bar 300 µm. Splenocytes were stained with anti-B220, GL-7, CD138 and PNA. Frequency of germinal center B cells is indicated. **(b)** 15 days after immunization PNA^{hi} B cells from ΔdbIGATA-1 were sorted and Vk-Ox1 V-region genes amplified and sequenced. The key mutations conferring high affinity are highlighted. (**c)** The percentage of CD138⁺ plasma cells was determined in the spleen of BALB/c and ΔdbIGATA-1 at day 0 and day 15 after primary immunization (contour plots). The percentage of CD138⁺ plasma cells in spleen during primary and secondary immunization was summerized from 4 animals per each group (graph). All error bars are s.d.

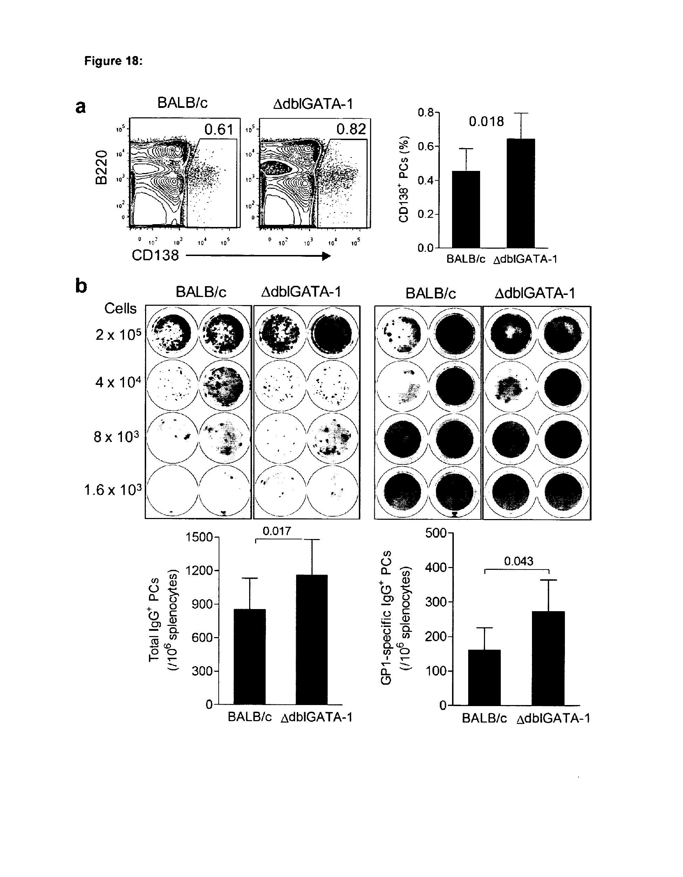

**Figure 18****: Eosinophil-deficiency affects numbers of plasma cells in spleen of LCMV infected mice.** BALB/c and ΔdbIGATA-1 mice were infected with LCMV and 7 months later the response analyzed. **(a)** The frequency of plasma cells in spleen was determined by flow cytometry. **(b)** Serial dilutions of splenocytes were analyzed by ELISPOT assays to determine the number of total (left panels) or GP1 (right panels) specific IgG plasma cells. Data from 6 animals for each group are summarized (graphs). P values are included, error bars display as s.d.

### EXAMPLES

The following examples represent experimental support for the invention as claimed and serve as a non-limiting description of the inventive concept of the invention. The present invention also relates to eosinophils as a target in the reduction of plasma cells in human subjects. The experiments described herein provide examples of the claimed invention in mice. Analogous experiments to those described in the examples carried out in humans and/or ex vivo human models are considered to exhibit the same effects and are included in the scope of the invention.

### Mo/Mφ and eosinophils produce APRIL

To determine which cells in the BM provide plasma cell survival factors, BM cell suspensions were prepared from naive BALB/c mice and analyzed by multi-parameter flow-cytometry. Staining with Gr-1 and F4/80 specific antibodies showed that in non-immunized BALB/c mice about 10% of BM cells are F4/80⁺Gr-1^{lo} cells, and these cells express APRIL, as revealed by intracellular staining **(****Fig. 1a, b**). Costaining of BM tissue sections with F4/80 and APRIL specific antibodies confirmed that all F4/80⁺Gr-1^{lo} cells expressed APRIL **(****Fig. 1b****, right panel).**

Side scatter (SSC) and CD11b expression revealed that F4/80⁺Gr-1^{lo} cells in the BM are a heterogeneous cell population **(****Fig. 1c****)**. Only about half of the F4/80⁺Gr-1^{lo}CD11b⁺ cells had low SSC as expected for Mo/Mφ **(****Fig. 1c****).** Cells with higher SSC (fractions R1 and R2) were also Siglec-F⁺, indicating that these cells are eosinophils **(****Fig. 1c****).** It is known that BM eosinophils express F4/80²³.

For further characterization we sorted the F4/80⁺Gr-1^{lo} R1, R2 and R3 cell fractions, as well as conventional DC (Gr-1^{lo/-}CD11c^{hi}), and neutrophils (Gr-1^{hi}) from BM and Mφ from the peritoneal cavity (PerC) **(****Fig. 8a, b, c**). Hematoxylin/eosin staining confirmed that cells in fraction R3 were Mo/Mφ (F4/80⁺Gr-1^{lo}CD11b^{int}Siglec-F⁻SSC^{lo}) **(****Fig. 1d****),** while R1 (F4/80⁺Gr-1^{lo}CD11b^{int}Siglec-F^{hi}SSC^{hi}) and R2 (F4/80⁺Gr-1^{lo}CD11b^{hi}Siglec-F⁺SSC^{int}) cells were eosinophils, as indicated by the strong eosin staining in the cytoplasm. The smaller size and the bi-lobed nucleus with compact chromatin identify R1 cells as mature eosinophils (Eos), whereas the larger size and the doughnut or ring-shaped nucleus with loose chromatin indicates R2 cells as immature eosinophils (EosP) **(****Fig. 1d****).** It demonstrates that in the BM both, Mo/Mφ and eosninophils produce APRIL.

### BM eosinophils produce plasma cell survival factors

To directly assess whether eosinophils are a major source of plasma cell survival factors, BM cells were stained for Siglec-F and for intracellular expression of APRIL and IL-6 **(****Fig. 1e****).** Siglec-F^{hi}Gr-1^{lo} eosinophils co-expressed high levels of APRIL and IL-6, whereas Siglec-F^{int} Gr-1^{lo} immature eosinophils expressed only APRIL. Siglec-F⁻Gr-1^{lo} Mo/Mφ expressed APRIL, however, only a small fraction of them, if any, expressed low levels of IL-6 as well (**Fig. 1e**). Gating on Gr-1^{lo}APRIL^{hi} or Gr-1^{lo}IL-6⁺ cells showed that the majority of APRIL^{hi} cells (fractions R1 and R2) and IL-6⁺ cells (fraction R1) are eosinophils (Fig. 1e). These findings were confirmed by staining cytospins of eosinophils (R1), immature eosinophils (R2) and Mo/Mφ (R3) with APRIL and IL-6 specific antibodies (Fig. 1f). The purity of the sorted populations was controlled by using antibodies specific for the major basic protein (MBP), a specific marker for eosinophils²⁴.

The expression of plasma cell survival factors in BM eosinophils was compared with that of Mo/Mφ, Gr-1^{hi} neutrophils and CD11c^{hi} conventional DC **(****Fig. 9****).** Real-time PCR showed that in the BM, APRIL and IL-6 mRNA expression was mainly seen in F4/80⁺Gr-1^{lo} eosinophils and Mo/Mφ and confirmed that eosinophil precursors do not express IL-6 **(****Fig. 9a****).** IL-6 mRNA expression corresponded with IL-6 secretion *in vitro,* where the highest expression was found in mature eosinophils (Fig. 9b). These data show that BM eosinophils are a major source of cytokines which are required for plasma cell survival.

### Eosinophils support plasma cell survival

To address whether eosinophils support survival of plasma cells, we co-cultured sorted BM eosinophils and plasma cells **(****Fig. 8b, d**). Eosinophils were prepared from non-immunized and immunized BALB/c mice. Injection of the T cell-dependent antigen 2-phenyl-oxazolone (phOx) increased the frequency of eosinophils in the BM **(****Fig. 10a****)** and induced an activated phenotype, as shown by their enhanced secretion of IL-4 **(****Fig. 2a****).** An even stronger activation was seen after secondary immunization with soluble phOx **(****Fig. 2a****).** Together with the enhanced expression of IL-4, APRIL and IL-6 mRNA and protein were also significantly higher **(****Fig. 2b****).** Similar effects were seen whether the antigen was delivered i.p. in Alum or emulsified in complete Freund's adjuvant (CFA) **(****Fig. 2a, b** and **Fig. 10a, b**).

Co-cultures of plasma cells and eosinophils isolated 6 days after secondary immunization showed that eosinophils support plasma cell survival in a dose-dependent manner **(****Fig. 2c****).** Plasma cells survival increased when eosinophils were isolated from the BM of primary immunized BALB/c mice, and even more following secondary immunization **(****Fig. 2d****).** The addition of the soluble fusion protein TACI-Ig²⁵, which blocks APRIL, or of neutralizing IL-6 antibodies to the culture medium significantly reduced the number of antibody-secreting cells **(****Fig. 2e****).** The strongest effect was seen when both inhibitors were added, suggesting that both APRIL and IL-6 are required for plasma cell survival and that both factors are provided by eosinophils.

In addition, approximately 90% of plasma cells are AnnexinV⁺ after 48 h of culture in the absence of eosinophils **(****Fig. 11 a)****.** Plasma cell apoptosis was prevented not only by direct contact with eosinophils but also by co-culture in a two-chamber transwell system (about 50% of plasma cells were Annexin-V) **(****Fig. 2f** and **Fig. 11a****).** Plasma cell survival was significantly reduced by the addition of TACI-Ig and anti IL-6 specific antibodies to the culture medium **(****Fig. 2g** and **Fig. 11b**), giving further evidence that eosinophils support plasma cell survival by providing soluble APRIL and IL-6. These data suggest that eosinophils support plasma cell survival by secretion of APRIL and IL-6.

### Eosinophils and plasma cells colocalize in the BM

To determine whether eosinophils and plasma cells interact in the BM, BALB/c mice were immunized with phOx and 6 days after secondary immunization, when the newly induced plasma blasts home to the BM, tissue sections were prepared. Staining with anti-Laminin, an antibody specific for the basement membrane protein of endothelial cells, showed that plasma cells localize along the thin-walled venous sinusoids **(****Fig. 3a****).** Co-staining with IgG- and APRIL-specific antibodies showed closely associated pairs of plasma cells and APRIL⁺ cells **(****Fig. 3a****).** The association between eosinophils and plasma cells was even more pronounced at day 60 after secondary immunization. Staining of BM tissue sections revealed large clusters containing multiple eosinophils and plasma cells **(****Fig. 3b****).** Triple staining for MBP and IgG together with APRIL or F4/80 revealed that 6 days after secondary immunization nearly 60% of plasma cells co-localize with APRIL⁺ cells **(****Fig. 3c****).** Approximately half of the plasma cells were involved in associations with eosinophils, and only 20% with macrophages **(****Fig. 3c****).** 60 days after secondary immunization the percentage of plasma cells in association with eosinophils was even higher **(****Fig. 3c****).**

To further characterize the cellular composition of the putative BM plasma cell survival niche, tissue sections were stained with VCAM-1 and MBP specific antibodies. VCAM-1 is expressed on stromal cells and also on a fraction of eosinophils **(****Fig. 3d** **left panel).** The analysis showed clustering of eosinophils in the underlying reticulum of stromal cells **(****Fig. 3d** **left panel).** Higher magnification showed that eosinophils were the major source of APRIL and IL-6 **(****Fig. 3d** **middle** and **right panels**). 60 days after secondary immunization more than 60% of BM plasma cells were found in close association with eosinophils and stromal cells **(****Fig. 3e****).** Both co-localization and the provision of APRIL and IL-6 suggest that eosinophils are an essential part of the plasma cell survival niche.

Plasma cells express the chemokine receptor CXCR4 and respond to its ligand CXCL12¹³. RT-PCR showed that neither Mo/Mφ nor eosinophils express CXCL12 **(****Fig. 12a****).** Based on this observation it is unlikely that these cells directly attract plasma cells. However, both Mo/Mφ and eosinophils expressed CXCR4 at the mRNA and at protein levels **(****Fig. 12b****).** The mean fluorescence intensity (MFI) for cell surface expression was most pronounced on mature eosinophils, while less intense staining was seen on eosinophil precursors and on Mo/Mφ **(****Fig. 12b****).** Thus plasma cells and mature eosinophils colocalize in the BM, maybe by being attracted to the same CXCL12-secreting sites.

### APRIL and IL-6 expression in eosinophil-deficient mice

To directly address whether eosinophils are required for the maintenance of plasma cells in the BM we analyzed mice which lack mature eosinophils²⁶. ΔdbIGATA-1 mice have a deletion in the high-affinity GATA-binding site of the GATA-1 promoter, which allows normal hematopoiesis, but specifically blocks the development of mature eosinophils. The frequency of F4/80⁺Gr-1^{lo} cells was reduced by about 50% in these mice in comparison to wild-type BALB/c mice **(****Fig. 4a****).** Staining for CD11b and Siglec-F demonstrated the specific absence of mature eosinophils **(****Fig. 4a****).** Furthermore, the absence of eosinophils in ΔdbIGATA-1 mice was confirmed by staining of BM tissue sections with MBP specific antibodies **(****Fig. 4b****).** Only a few weakly MBP-positive cells, presumably eosinophil precursors, were observed.

In contrast to wild-type BALB/c mice, APRIL expression was low in the BM of ΔdbIGATA-1 mice **(****Fig. 4b****)** and, in line with these observations, the level of APRIL mRNA was significantly reduced **(****Fig. 4c****).** The weak APRIL staining was restricted to Mo/Mφ and endothelial cells. In addition, in eosinophil-deficient mice the number of IL-6 expressing cells was reduced **(****Fig. 4b****).** Accordingly, the level of IL-6 mRNA extracted from total BM of ΔdbIGATA-1 mice cells was significantly lower than in BALB/c mice and when BM cells from eosinophil-deficient mice were cultured *in vitro,* IL-6 secretion was hardly detectable **(****Fig. 4c****).**

Similar results were found in PHIL mice, another mouse strain which lacks mature eosinophils²⁷. In these animals expression of the cytocidal diphteria toxin A chain under the control of the eosinophil peroxidase promoter prevents development of mature eosinophils²⁷. A clear reduction in APRIL and IL-6 expression was seen in the BM these mice, with the remaining APRIL expression restricted to Mo/Mφ **(****Fig. 13****).** These data indicate that mature eosinophils are the major source of the plasma cell survival factors APRIL and IL-6 in the BM.

### Steady-state plasma cells maintenance in eosinophil-deficient mice

We investigated whether the absence of eosinophils affects the number of plasma cells in BM. The number of plasma cells was reduced in the BM of ΔdbIGATA-1 mice at steady state **(****Fig. 5a****).** The frequency of CD138⁺ plasma cells **(****Fig. 5a****, left panel),** as well as the absolute number of IgG-positive plasma cells **(****Fig. 5a****, middle panel)** was reduced to one third, as revealed by flow cytometry and ELISPOT assays. These observations correlated with a significant reduction in total IgG secretion from cultured BM cells from ΔdbIGATA-1 mice **(****Fig. 5a****, right panel).** Staining of BM tissue sections gave further evidence that the number of plasma cells in the BM of ΔdbIGATA-1 was significantly lower than in wild-type BALB/c mice **(****Fig. 14a****).** A significant reduction of BM plasma cell numbers was seen at steady state in PHIL mice as well **(****Fig. 14a****).**

ΔdbIGATA-1 mice show normal development of B cells in the BM **(****Fig. 15a****)** and normal differentiation into newly formed T1 and T2, marginal zone and follicular B cells **(****Fig. 15b****).** Furthermore, laminin and VCAM-1 staining of BM sections from ΔdbIGATA-1 mice showed the typical organization of the BM structure and normal development of the venous and arterial sinus architecture **(****Fig. 16a****).** The level of CXCL12 mRNA expression in total BM cells suggested that stromal cells express normal levels of chemokines **(****Fig. 16b****).** Although eosinophil- deficiency does not effect the development and the organization of the BM, the maintenance of plasma cells was impaired at steady state.

### Plasma cell accumulation in eosinophil-deficient mice

Next we investigated the maintenance of plasma cells in BM and spleen after immunization with the T_{H}2 antigen phOx. To exclude a general defect in the generation of antigen-specific plasma cells, the splenic immune response in ΔdbIGATA-1 mice was analyzed. ΔdbIGATA-1 responded with normal germinal center development **(****Fig. 17a****).** As described for wild-type BALB/c mice, antibodies with a Vk-Ox1 L-chain dominated the phOx specific immune response²⁸⁻²⁹. Sequencing showed normal accumulation of somatic mutations and selection for high affinity variants **(****Fig. 17b****).** Furthermore, the frequency of splenic plasma cells was comparable to that in BALB/c mice **(****Fig. 17c****).** Nevertheless, the frequency and the numbers of plasma cells in BM of ΔdbIGATA-1 mice were significantly reduced **(****Fig. 5b** and **Fig. 14b****).**

Upon secondary immunization, a clearly detectable influx of antigen-specific plasma cells into the BM was observed **(****Fig. 5b****)** which is in line with the normal expression levels of CXCR4 on splenic plasma cells **(****Fig. 16c****).** The overall kinetics for the increase and decrease of plasma cell numbers in the BM were similar between ΔdbIGATA-1 and wild-type BALB/c mice. The highest number of total and antigen-specific plasma cells was detected 6 days after antigen boost, followed by a steep decline **(****Fig. 5b** and **Fig. 14b****).** In eosinophil-deficient ΔdbIGATA-1 mice, the number of antigen-specific plasma cells in the BM 12 days after secondary immunization was no longer significantly different from the number before boost **(****Fig. 5c****).** In contrast, even 60 days after booster injection of antigen, the frequency and absolute number of antigen-specific plasma cells remained significantly increased in the BM of immunized wild-type mice **(****Fig. 5b, c****).**

As eosinophils are thought to be mainly associated with T_{H}2 immune responses³⁰, we asked whether the survival of plasma cells in the BM is affected by the absence of eosinophils in a T_{H}1 response. BALB/c and ΔdbIGATA-1 mice were infected with lymphocytic choriomeningitis virus (LCMV), which induces a strong T_{H}1 response³¹. Eosinophil-deficient ΔdbIGATA-1 mice responded as well as BALB/c mice to LCMV **(****Fig. 18****).** Nevertheless, the frequency of plasma cells in the BM of ΔdbIGATA-1 mice was significantly reduced **(****Fig. 5d****).** The reduction was seen in the total number of IgG⁺ plasma cells (data not shown) and in the number of glycoprotein 1 (GP1, antigen of LCMV)-specific plasma cells **(****Fig. 5e****).** These results show that eosinophils are required for the maintenance of plasma cells in the BM, in both T_{H}1 and T_{H}2 dependent immune responses.

### Eosinophil dependent BM retention of plasma cells

Reconstitution experiments were set up to directly investigate whether the retention of plasma cells in the BM requires the presence of eosinophils. Six days after secondary immunization of BALB/c mice, BM eosinophils were sorted and transferred into recipient ΔdbIGATA-1 mice. BM analysis of reconstituted ΔdbIGATA-1 showed that transferred eosinophils can migrate back into the BM, however, only about one third of them survived for 12 days post transfer **(****Fig. 6a, b****)**.

Eosinophil reconstitution of immunized ΔdbIGATA-1 mice affected the number of plasma cells in BM and spleen **(****Fig. 6c, d****).** 6 days after secondary immunization the presence of eosinophils was sufficient to significantly increase the number of antigen-specific plasma cells in the BM of ΔdbIGATA-1 mice **(****Fig. 6c****)** and concomitantly to decrease them in the spleen to nearly normal levels **(****Fig. 6d****).** However, the effect was transient, as the number of plasma cells in the BM of reconstituted mice was comparable to that of non-reconstituted ΔdbIGATA-1 mice 12 days after secondary immunization, when transferred eosinophils numbers had declined. These data demonstrate a direct role of eosinophils for the retention of plasma cells in the BM.

### Eosinophils dependent long-term survival of plasma cells

To answer the question whether eosinophils are required for the long-term survival of plasma cells, BALB/c mice were immunized with phOx. 60 days after secondary immunization long- lived antigen-specific plasma cells are mainly found in the BM⁷. Injection with a Siglec-F specific antibody, which is known to reduce the number of eosinophils³², resulted in nearly complete depletion of eosinophils in blood, spleen and BM of BALB/c mice 4 days after the last injection **(****Fig. 7a****).** Depletion of eosinophils induced a significant reduction in the frequency of total plasma cells and the number of antigen-specific plasma cells **(****Fig. 7b** and **7c****).** One week after anti-Siglec-F injection the frequency of BM plasma cells was reduced by 50%. On average, 16% of the remaining BM plasma cells were Annexin-V⁺ **(****Fig. 7d****),** suggesting that, in the absence of eosinophils, plasma cells undergo apoptosis. A reduction in the number of plasma cell following depletion of eosinophils was only observed in the BM. No effect on the number of plasma cells **(****Fig. 7b** and **7c****)** and no increase in the frequency of Annexin-V⁺ plasma cells **(****Fig. 7d****)** were observed in the spleen. These data demonstrate that eosinophils have a pivotal role for the long-term survival of plasma cells in the BM. As the main source of plasma cell survival factors, eosinophils are an essential part of the plasma cell survival niche.

### Materials and Methods used in the examples of the invention

**Mice:** BALB/c mice were purchased from Charles River, ΔdbIGATA-1 mice (BALB/c background) from The Jackson Laboratory, PHIL (C57BL/6 background) and C57BL/6 mice from Mayo Clinic Arizona, US. For primary immunization, mice were immunized i.p. with 100µg of alum-precipitated or CFA-emulsified phOx coupled to CSA. After 6-8 weeks animals were boosted i.v. with 100µg soluble antigen. BALB/c and ΔdbIGATA-1 mice were infected with 2x10⁶ pfu of LCMV strain WE and 7 months later analyzed. Animal experiments were approved by the animal care and use committee (Landesamt für Gesundheit und Soziales, Berlin).

**Antibodies and reagents:** Anti-CD11b (M1/70); anti-CD11c (N418), anti-Ly6G (Gr-1, RB6-8C5); anti-F4/80; anti-VCAM-1 (6C71); anti-IL-6 (MP5-20F3); anti-B220 (RA3.6B2); anti-IgD (11.26c); anti-GL-7; anti-IgM (M41); anti-CD4 (GK1.5); anti-CD21 (7G.6) were supplied from DRFZ. Anti-Siglec-F (E50-2440); anti-CD138 (281-2); anti-CD23 (B3B4); anti-CXCR4 (2B11/CXCR4), and Annexin-V were purchased from BD Pharmingen. Anti-FcεRIα was ordered from eBioscience, anti-APRIL from Stressgen, anti-MOMA-1 from BMA Biomedicals, PNA from Vector. As isotype controls rat IgG2a (KLH/G2a-1-1), IgG2b (KLH/G2b-1-2), IgG1 (KLH/G1-2-2) and rabbit IgG (Southern Biotech), anti-mouse IgG (Molecular Probes). Rabbit anti-mouse Laminin was purchased from Sigma, monoclonal rat anti-mouse MBP from NA Lee and JJ Lee, Mayo Clinic Arizona, US. Fluorescent dyes conjugated secondary goat-anti rabbit IgG, anti-rat IgG (Molecular Probes), Streptavidin (Molecular Probes or BD) and anti-Dig (DRFZ) were used. Cell suspensions from spleen or BM were stained for surface and intracellular expression as previously described²⁵. Stained cells were analyzed by LSRII and data analyzed using FlowJo.

**Cell preparation and cell culture:** Single cell suspensions from BM and spleen were prepared. To isolate BM DC, cells were stained with anti-CD11c-PE, incubated with anti-PE beads, enriched by MACS and sorted by FACS. To sort neutrophils the CD11c negative fraction was stained with anti-Gr-1 and anti-CD11b **(****Fig. 8a****).** To isolate eosinophils (R1), immature eosinophils (R2) and Mo/Mφ (R3), BM cell suspension was depleted of B and T cells by MACS and cells stained with anti-CD11c/FcεRIα, anti-Gr-1, anti-F4/80 and anti-CD11b. BM DC, basophils/mast cells and neutrophils were excluded by gating. The R1, R2 and R3 cell subsets were sorted based on SSC and CD11b **(****Fig. 8b****).** Peritoneal cavity cells were plated in RPMI 1640 medium supplemented with 10% FCS (GIBCO), 50 µM 2-ME (Sigma), 100 U/ml penicillin, and 100 µg/ml streptomycin in 6 cm plastic dishes (Costar) for 2 hours. Non-adherent cells were removed by washing 4 times with PBS and adherent Mφ isolated **(****Fig. 8c****).** Total BM (10⁶cells/ml) or sorted BM (2x10⁵ cells/200µl) cell populations were cultured in supplemented RPMI 1640 medium for 24h. Secretion of cytokines was measured by Bio-plex (Biorad), total IgG and antigen specific IgG concentrations by standard ELISA.

**Cytospins and immunostaining:** For cytospins, 1x10⁵ sorted BM cells in 150µl complete medium were deposited into 48-well plates (Costar) containing poly-L-lysine-coated 12mm round cover slips (BD). After 3 hours plates were centrifuged, washed with PBS and fixed with 100% cold ethanol for 10 min.

Femurs and tibias were carefully flushed with DMEM (GIBCO), the intact BM and spleen frozen immediately in Tissue-Tec OCT compound and stored at -70°C. Tissue sections of 5-7 µm were prepared and used for fluorescence or immunohistochemical staining as indicated.

**The analysis of the immune response:** BALB/c and ΔdbIGATA-1 mice were immunized with phOx. Germinal center B cells (GL7⁺PNA^{hi}) were sorted at day 15 and VₖOx1 genes sequenced²⁸. ELISPOT (Immunspot Analyzer, CTL), was used to determine the number plasma cells. 2x10⁵ spleen or BM cells were 5 fold diluted and incubated 3h on anti-mouse Ig, phOx-CSA or GP1 coated plates (Millipore). After incubation with secondary antibody (Southern Biotech) the number of spots was counted, the Ig titer measured by using standard ELISA.

**Gene expression analysis:** Total RNA was extracted from 5x10⁵-10⁶ cells using NucleoSpin^{®}RNA II (Macherey-Nagel) and reverse transcribed into cDNA using a Sensiscript RT kit (Qiagen). The levels of APRIL, IL-6, CXCR4 and CXCL12 expression were determined by RT-PCR and real-time PCR²⁵. Primer sequences (TibMolBiol) are listed in **Table 1.**

**Co-culture of plasma cells and eosinophils:** BALB/c mice were immunized with phOx and 6 days after boosting, BM CD138⁺ plasma cells were purified using an isolation kit (Miltenyi Biotec) **(****Fig. 8d****).** 100 plasma cells were cultured with eosinophils in U bottom 96 well plates. After 24h coculturing, cells were transfered to anti-mouse Ig-coated plates and incubated at 37°C for additional 24h. The number of spots was counted by ELISPOT. Furthermore, 10⁴ plasma cells were cultured together with eosinophils separately in transwells (5µm pore size) (Costar) for 48h and the percentage of Annexin-V⁺ cells determined. In blocking experiments the soluble fusion protein TACI-Ig, anti IL-6 antibodies (RD) or both were added. As isotype control human IgG or rat IgG1 was used.

**Adoptive transfer of eosinophils:** 6 days after antigen boost BM cells from BALB/c mice were depleted by MACS sort using a coctail of antibodies (biotinylated anti-B220, anti-IgM, anti-CD4 and anti-CD8) and eosinophils sorted as described in **Fig 8****.** 10⁷ sorted eosinophils per day were transferred into ΔdbIGATA-1 mice by i.v. injection at 2 consecutive days and 6 or 12 days later the number and percentage of eosinophils in BM was measured. To investigate whether reconstitution with eosinophils rescues the accumulation of plasma cells, BALB/c and ΔdbIGATA-1 mice were immunized with phOx-CSA in Alum and 6 weeks later ΔdbIGATA-1 mice were reconstituted with eosinophils (daily i.v. injections of 1x10⁷ eosinophils between days -2 and +1). At day 0 mice were boosted with soluble antigen and the number and percentage of plasma cells determined at days 6 and 12.

**Eosinophil depletion:** BALB/c mice were injected three times with 20µg soluble anti-mouse Siglec-F (i.p, every second day). Rat IgG2a was used as an isotype-matched Ab (R&D systems). 4 days after the last injection, eosinophils were analyzed in BM, blood and spleen.

**Statistical analysis:** For statistical analysis a paired two-tailed Student't-test and Two-way ANOVA was performed assuming equal variance of samples. Error bars represent standard deviation (s.d.), *P* values are indicated.

**Table 1: List of PCR primer sequences**

| **Gene** | **5' Primer sequence** | **3' Primer sequence** |
|---|---|---|
| CXCL12 | 5'-ggacgccaaggtcgtcgccgtgctg | 5'-ctccaggtactcttggatccac |
| CXCR4 | 5'-gccatggaaccgatcagtgtgag | 5'-ccatgaccaggatcaccaatcc |
| APRIL | 5'-gcaaccagtacttaggcgtgg | 5'-aggcacggtcaggatcagaag |
| IL-6 | 5'-ccacttcacaagtcggaggct | 5'-ggtactccagaagaccagagg |
| ß-actin | 5'-gacaggatgcagaaggagatcact | 5'-tgatccacatctgctggaggt |

### DISCUSSION

The BM provides a micro-environment which supports the long-term maintenance of plasma cells by providing survival factors such as APRIL and IL-6¹⁶⁻¹⁸. The invention shows that F4/80⁺Gr-1^{lo} eosinophils are the main source for these survival factors in the BM. *In vitro* co-culturing showed that eosinophils support plasma cell survival by secreting APRIL and IL-6. The analysis of eosinophil-deficient mice revealed that in the absence of eosinophils the maintenance of plasma cell in the BM is impaired. We demonstrate that eosinophils are required both for plasma cell retention and also for the long-term survival of these cells in the BM. Thus, our data demonstrate eosinophils as central players in the long-term maintenance of immune protection.

Expression of CXCR4 on mature eosinophils and plasma cells may confer similar homing properties to both cell types, resulting in their co-localization to sites of CXCL12 secretion. During hematopoiesis CXCR4 is required for the retention of eosinophil precursors in the BM³³⁻³⁴ and during immune responses CXCL12 secreted by BM stromal cells may guide resident eosinophils into the plasma cell survival niche¹²⁻¹³. Although both eosinophils and Mo/Mφ express CXCR4, we find that mainly eosinophils co-localize with plasma cells. Less pronounced surface expression of CXCR4 on Mo/Mφ may explain this preferential association of eosinophils with plasma cells.

The importance of eosinophils for the long-term maintenance of plasma cells in the BM was tested in ΔdbIGATA-1 mice, a mouse strain with normal immune structures, but lacking mature eosinophils. Alum-precipitated phOx antigen was used for primary immunization as this adjuvant induces an antigen-independent activation of eosinophils measurable as an enhanced secretion of IL-4³⁵⁻³⁶. Activated eosinophils promote the early expansion and differentiation of antigen-specific B cells into IgM-secreting plasma cells³⁵. However, upon secondary immunization with soluble phOx, an even stronger activation was seen, and enhanced IL-4 secretion by eosinophils was accompanied by increased expression of the plasma cell survival factors APRIL and IL-6. This likely accounts for the improved capacity of eosinophils isolated from secondary immunized animals to prolong plasma cell survival.

The fact that immunization of ΔdbIGATA-1 mice with the T cell-dependent antigen phOx or infection with LCMV induces normal antigen-specific immune responses supports the notion that eosinophils are not required for the induction of either T_{H}2 or T_{H}1 responses. However, eosinophils are needed for the retention and long-term maintenance of plasma blasts in the BM. Our data suggest that eosinophils are required as a continuous source of survival factors. As soon as eosinophils are depleted, plasma cells are rapidly dying by apoptosis. Once plasma blasts have settled in the BM and developed into mature plasma cells they are apparently unable to leave the BM. In line with this interpretation we see no significant increase in the number of plasma cells in the spleen of BALB/c mice depleted from eosinophils for one week. A re-location of plasma cells to the spleen was only observed at the early phase of the immune response, when the newly generated plasma blasts where unable to accumulate in the BM of eosinophil-deficient ΔdbIGATA-1 mice. While eosinophils seem to provide a constitutive source of plasma cell survival factors in the BM, DCs and Mo/Mφ together with neutrophils and epithelial cells may provide sufficient APRIL and IL-6 to support the maintenance of plasma cells at peripheral sites, such as lymph nodes and lamina propria^{20, 37-38}.

Eosinophils are of importance in human immune responses. One indication comes from a report describing a patient with common variant immune deficiency who lacked eosinophils and had remarkably few plasma cells in the BM³⁹. This finding supports the notion that, like their murine counterparts, human eosinophils play a major role in the survival of plasma cells. Eosinophils have an important role in chronic asthmatic inflammation, being critically involved in T cell activation in allergic inflammatory responses both as antigen-presenting cell and also as a major source of T_{H}2 cytokines such as IL-4^{30, 40}. In allergic or asthmatic situations eosinophils, attracted into the inflamed mucosal tissues, may not only support the T_{H}2 mediated inflammatory process but also prolong the ectopic maintenance of plasma cells and thus further enhance the allergen-specific immune responses. As our data show that eosinophils are required for the long-term survival of plasma cells, eosinophil-directed therapy provide a novel therapeutic approach to treat autoimmune and other diseases where plasma cells contribute to the pathogenesis.

### REFERENCES

1. Ahmed, R. & Gray, D. Immunological memory and protective immunity: understanding their relation. Science 272, 54-60 (1996).
2. Mamani-Matsuda, M. et al. The human spleen is a major reservoir for long-lived vaccinia virus-specific memory B cells. Blood 111, 4653-4659 (2008).
3. Manz, R.A., Hauser, A.E., Hiepe, F. & Radbruch, A. Maintenance of serum antibody levels. Annu Rev Immunol 23, 367-386 (2005).
4. Fairfax, K.A., Kallies, A., Nutt, S.L. & Tarlinton, D.M. Plasma cell development: from B-cell subsets to long-term survival niches. Semin Immunol 20, 49-58 (2008).
5. Smith, K.G., Light, A., Nossal, G.J. & Tarlinton, D.M. The extent of affinity maturation differs between the memory and antibody-forming cell compartments in the primary immune response. EMBO J 16, 2996-3006 (1997).
6. Shapiro-Shelef, M. & Calame, K. Regulation of plasma-cell development. Nat Rev Immunol 5, 230-242 (2005).
7. Radbruch, A. et al. Competence and competition: the challenge of becoming a long-lived plasma cell. Nat Rev Immunol 6, 741-750 (2006).
8. Blink, E.J. et al. Early appearance of germinal center-derived memory B cells and plasma cells in blood after primary immunization. J Exp Med 201, 545-554 (2005).
9. Dilosa, R.M., Maeda, K., Masuda, A., Szakal, A.K. & Tew, J.G. Germinal center B cells and antibody production in the bone marrow. J Immunol 146, 4071-4077 (1991).
10. Benner, R., Hijmans, W. & Haaijman, J.J. The bone marrow: the major source of serum immunoglobulins, but still a neglected site of antibody formation. Clin Exp Immunol 46, 1-8(1981).
11. Minges Wols, H.A., Underhill, G.H., Kansas, G.S. & Witte, P.L. The role of bone marrow-derived stromal cells in the maintenance of plasma cell longevity. J Immunol 169, 4213-4221 (2002).
12. Tokoyoda, K., Egawa, T., Sugiyama, T., Choi, B.I. & Nagasawa, T. Cellular niches controlling B lymphocyte behavior within bone marrow during development. Immunity 20, 707-718 (2004).
13. Hargreaves, D.C. et al. A coordinated change in chemokine responsiveness guides plasma cell movements. J Exp Med 194, 45-56 (2001).
14. Hauser, A.E. et al. Chemotactic responsiveness toward ligands for CXCR3 and CXCR4 is regulated on plasma blasts during the time course of a memory immune response. J Immuno/ 169, 1277-1282 (2002).
15. Nie, Y. et al. The role of CXCR4 in maintaining peripheral B cell compartments and humoral immunity. J Exp Med 200, 1145-1156 (2004).
16. Cassese, G. et al. Plasma cell survival is mediated by synergistic effects of cytokines and adhesion-dependent signals. J Immunol 171, 1684-1690 (2003).
17. Belnoue, E. et al. APRIL is critical for plasmablast survival in the bone marrow and poorly expressed by early-life bone marrow stromal cells. Blood 111, 2755-2764 (2008).
18. O'Connor, B.P. et al. BCMA is essential for the survival of long-lived bone marrow plasma cells. J Exp Med 199, 91-98 (2004).
19. Lamb, R.J., Capocasale, R.J., Duffy, K.E., Sarisky, R.T. & Mbow, M.L. Identification and characterization of novel bone marrow myeloid DEC205+Gr-1+ cell subsets that differentially express chemokine and TLRs. J Immunol 178, 7833-7839 (2007).
20. Huard, B. et al. APRIL secreted by neutrophils binds to heparan sulfate proteoglycans to create plasma cell niches in human mucosa. J Clin Invest 118, 2887-2895 (2008).
21. Mhawech-Fauceglia, P. et al. The source of APRIL up-regulation in human solid tumor lesions. J Leukoc Biol 80, 697-704 (2006).
22. Mohr, E. et al. Dendritic cells and monocyte/macrophages that create the IL-6/APRIL-rich lymph node microenvironments where plasmablasts mature. J Immunol 182, 2113-2123 (2009).
23. McGarry, M.P. & Stewart, C.C. Murine eosinophil granulocytes bind the murine macrophage-monocyte specific monoclonal antibody F4/80. J Leukoc Biol 50, 471-478 (1991).
24. Lee, J.J. et al. Interleukin-5 expression in the lung epithelium of transgenic mice leads to pulmonary changes pathognomonic of asthma. J Exp Med 185, 2143-2156 (1997).
25. Chu, V.T., Enghard, P., Riemekasten, G. & Berek, C. In vitro and in vivo activation induces BAFF and APRIL expression in B cells. J Immunol 179, 5947-5957 (2007).
26. Yu, C. et al. Targeted deletion of a high-affinity GATA-binding site in the GATA-1 promoter leads to selective loss of the eosinophil lineage in vivo. J Exp Med 195, 1387-1395 (2002).
27. Lee, J.J. et al. Defining a link with asthma in mice congenitally deficient in eosinophils. Science 305, 1773-1776 (2004).
28. Berek, C., Berger, A. & Apel, M. Maturation of the immune response in germinal centers. Cell 67, 1121-1129 (1991).
29. Berek, C. & Milstein, C. Mutation drift and repertoire shift in the maturation of the immune response. Immunol Rev 96, 23-41 (1987).
30. Spencer, L.A. & Weller, P.F. Eosinophils and Th2 immunity: contemporary insights. Immunol Cell Biol 88, 250-256 (2010).
31. Lohning, M. et al. Long-lived virus-reactive memory T cells generated from purified cytokine-secreting T helper type 1 and type 2 effectors. J Exp Med 205, 53-61 (2008).
32. Zimmermann, N. et al. Siglec-F antibody administration to mice selectively reduces blood and tissue eosinophils. Allergy 63, 1156-1163 (2008).
33. Nagase, H. et al. Expression of CXCR4 in eosinophils: functional analyses and cytokine-mediated regulation. J Immunol 164, 5935-5943 (2000).
34. Ma, Q., Jones, D. & Springer, T.A. The chemokine receptor CXCR4 is required for the retention of B lineage and granulocytic precursors within the bone marrow microenvironment. Immunity 10, 463-471 (1999).
35. Jordan, M.B., Mills, D.M., Kappler, J., Marrack, P. & Cambier, J.C. Promotion of B cell immune responses via an alum-induced myeloid cell population. Science 304, 1808-1810 (2004).
36. McKee, A.S. et al. Alum induces innate immune responses through macrophage and mast cell sensors, but these sensors are not required for alum to act as an adjuvant for specific immunity. J Immunol 183, 4403-4414 (2009).
37. He, B. et al. Intestinal Bacteria Induce T Cell-Independent IgA2 Class Switching by Linking Epithelial Cells with Lamina Propria B Cells through APRIL. J Immunol 178, S28-(2007).
38. Cerutti, A. Location, location, location: B-cell differentiation in the gut lamina propria. Mucosal Immunol 1, 8-10 (2008).
39. Juhlin, L. & Michaelsson, G. A new syndrome characterised by absence of eosinophils and basophils. Lancet 1, 1233-1235 (1977).
40. Hogan, S.P. Recent advances in eosinophil biology. Int Arch Allergy Immunol 143 Suppl 1, 3-14 (2007).

## Claims

1. Method for the modulation of plasma cell function and/or number by the modulation of eosinophil number and/or function.

2. Method according to claim 1, **characterised in that**
plasma cells are depleted by depletion and/or functional inhibition of eosinophils.

3. Method according to any one of the preceding claims, **characterised in that** the plasma cells and/or eosinophils are depleted via induction of apoptosis.

4. Method according to any one of the preceding claims, **characterised in that** IL-5, APRIL and/or IL-6 function, production and/or expression is inhibited.

5. Method according to claim 1, **characterised in that**
plasma cell function, numbers and/or development are induced and/or maintained by induction and/or functional activation of eosinophils.

6. Method according to the preceding claim, **characterised in that** IL-5, APRIL and/or IL-6 function, production and/or expression is induced and/or activated.

7. Method according to any one of the preceding claims, **characterised in that** the plasma cells are antigen-specific plasma cells, preferably of the bone-marrow, epithelial, lung and/or intestinal tissues.

8. Eosinophil-modulating agent for use as a medicament for the modulation of plasma cell number and/or function.

9. Eosinophil-modulating agent according to the preceding claim, **characterised in that** the agent is an anti-eosinophil agent for use as a medicament for the depletion of plasma cells, preferably by induction of apoptosis.

10. Anti-eosinophil agent according to the preceding claim for use as a medicament in the treatment of diseases **characterised by** an unwanted immune response.

11. Anti-eosinophil agent according to any one of claims 9 or 10, whereby the disease is **characterised by** an unwanted humoral immune response, preferably dependent on the numbers and/or activity of plasma cells, such as allergic reactions, autoimmune diseases and rejection of organs and/or tissue after transplantation.

12. Anti-eosinophil agent according to any one of claims 9 to 11 for application in the lung, preferably in mucosal tissues of the lung, in the treatment of an allergic reaction, such as asthma.

13. Anti-eosinophil agent according to any one of claims 9 to 14, **characterised in that** the agent comprises one or more compounds that target eosinophils, specific antigens of eosinophils and/or molecules and/or signalling pathways important in eosinophil survival and/or development, such as Siglec-8 and/or IL-5.

14. Eosinophil-modulating agent according to claim 8, **characterised in that** the agent is an eosinophil-stimulating agent, such as IL-5, for use as a medicament for the induction and/or maintenance of plasma cells.

15. Method for determining the presence and/or numbers of plasma cells in a sample by measuring the presence and/or numbers of eosinophil cell numbers, whereby the presence and/or numbers of eosinophils is positively correlated with the presence and/or numbers of plasma cells.

16. Anti-eosinophil agent according to any one of claims 9 to 13 for reducing levels of IgE antibodies in the treatment of an unwanted inflammatory response.

17. Anti-eosinophil agent according to any one of claims 9 to 13 or 16, **characterised in that** the agent prevents eosinophil development and/or facilitates depletion and/or inhibition of eosinophil function.

18. Eosinophil-modulating agent according to any one of claims 8 to 14, 16 or 17, **characterised in that** the agent is an antibody, small molecule, anti-sense nucleic acid molecule, such as siRNA, a cytokine and/or a peptide.

19. Pharmaceutical composition comprising an eosinophil-modulating agent with a pharmaceutically acceptable carrier.

20. Pharmaceutical composition according to the preceding claim comprising an anti-eosinophil agent according to any one of claims 9 to 13, 16 or 17, comprising preferably antibodies directed against Siglec-8 and/or IL-5, more preferably a combination of multiple antibodies directed against Siglec-8 and IL-5, with a pharmaceutically acceptable carrier.

21. Method for the treatment of an unwanted immune response, preferably an unwanted humoral immune response dependent on the numbers and/or activity of plasma cells, such as allergic reactions, autoimmune diseases and rejection of organs and/or tissue after transplantation, by administration of an anti-eosinophil agent according to any one of claims 9 to 13, 16 or 17, to a subject, **characterized in that** plasma cells are depleted in the subject.
